# EUROPEAN PATENT APPLICATION

(11) **EP 1 576 953 A1**
(43) Date of publication of application: **21.09.2005**
(21) Application number: 03777229.0
(22) Date of filing: 04.12.2003
(51) Int. Cl.: A61K 31/05, A61K 9/10, A61K 47/12, A61K 47/24, A61P 23/00, A61P 25/20

(54) **PROPOFOL-CONTAINING FAT EMULSIONS**

(30) Priority: 06.12.2002 JP 2002355880
(71) Applicant: OTSUKA PHARMACEUTICAL FACTORY, INC., Naruto-shi, Tokushima 772-8601 (JP)
(72) Inventor: MATSUDA, Kenji Green Shato Rapyuta 105, Itano-gun, Tokushima 771-0218 (JP); TAKEDA, Koichi, Itano-gun, Tokushima 771-0204 (JP); TERAO, Toshimitsu, Naruto-shi, Tokushima 772-0015 (JP); INOUE, Tadaaki, Tokushima-shi, Tokushima 770-0021 (JP)
(74) Representative: HOFFMANN EITLE
(86) International application number: PCT/JP2003/015510
(87) International publication number: WO 2004/052354

(57) **Abstract**

The present invention provides a propofol-containing fat emulsion comprising propofol, an oily component, and an emulsifier, and further comprising a predetermined amount of a stabilizer, such as phosphatidylglycerol in which a specific fatty acid is a constituent fatty acid component. The present invention also provides a pain-relieving propofol-containing fat emulsion, which is obtained by mixing a local anaesthetic in advance with the above-described propofol-containing fat emulsion of the invention.

## Description

### TECHNICAL FIELD

The present invention relates to propofol-containing fat emulsions.

### BACKGROUND ART

Propofol (2,6-diisopropylphenol) is a lipophilic substance which induces hypnosis, and is known as an effective component in medications such as general anesthetics, sedatives and the like. Usually, propofol is prepared into a pharmaceutical formulation in an oil-in-water fat emulsion using an oily component and emulsifier in such a manner that the propofol can be administered directly into the bloodstream either by intravenous injection or by infusion. Propofol in the form of the fat emulsion is widely used as a general anesthetic, sedative, etc. (U.S. Patent No. 5,714,520).

It has been reported that a strong sharp pain (vascular pain) often develops with a high frequency as a side effect during the administration of such preparations by intravenous injection or infusion (W. Klemment, J.O. Arndt: British Journal of Anaesthesia, 1991; 67; 281 -284).

This problem can be solved by incorporating into the fat emulsion a local anaesthetic, such as lidocaine, in an amount sufficient to remove pain. However, the prior-art propofol-containing fat emulsion has the disadvantage that it cannot be administered by injection or infusion when a local anaesthetic, such as lidocaine, is mixed therewith because the stability of the resulting emulsion is rapidly lost; emulsion particles agglomerate in a short time, usually within 30 minutes; and the emulsion breaks down, which causes a separation of water and oil phases (E.E.M. Lilley et al., Anaesthesia, 1996; 51: 815-818).

To overcome this serious drawback, i.e., the sharply reduced emulsion stability caused by the incorporation of lidocaine, etc., a technique has been proposed in which a hydrophilic surfactant having an HLB value of 10 or more, for example, polyoxyethylene (60) hydrogenated castor oil, etc., is incorporated as a stabilizer in the aqueous phase of a propofol-containing fat emulsion, and the pH thereof is adjusted within the range of 3.0 to 6.5 (Japanese Unexamined Patent Publication No. 2002-179562). In the fat emulsion obtained according to the proposed technique, the emulsion breakdown can be suppressed to some extent, however the technique has the disadvantage that the safety of the fat emulsion obtained is reduced because the safety of the hydrophilic surfactant that is used is low. Sterilization is essential not only for fat emulsions but also for other pharmaceutical preparations to assure their safety when they are commercially produced. Research by the inventors has shown that the above-proposed fat emulsions cannot be administered by injection or infusion under certain conditions because their aqueous phase and oil phase are separated when a standard sterilization method, such as high-pressure steam sterilization, is carried out. Thus, the proposed fat emulsions have the drawback that it cannot be sterilized by standard sterilization method.

### DISCLOSURE OF THE INVENTION

An object of the present invention is to provide a propofol-containing fat emulsion having high emulsion stability, with which a local anaesthetic, such as lidocaine or the like, can be mixed in advance, or before use, so as to prevent sharp pain (vascular pain) from occurring or alleviate the pain as a side effect during the administration thereof.

The present inventors carried out extensive research and found that propofol-containing fat emulsions with high emulsion stability can be obtained by the use of a specific phospholipid, a phospholipid derivative, or a fatty acid as a stabilizer in place of a hydrophilic surfactant having an HLB value of 10 or more, as was proposed for use as a stabilizer in the prior art. More specifically, the inventors found the following: the above-mentioned stabilizers have each excellent safety; fat emulsions obtained by the addition of such stabilizers are imparted excellent emulsion stability; and the emulsion stability is retained even when it is mixed with a local anaesthetic in an amount sufficient to relieve pain. The invention has been accomplished based on these findings and further research.

The present invention provides the inventions defined in Items 1 to 34:
Item 1. A fat emulsion with which a local anaesthetic is mixed before use, and which comprises propofol, an oily component, and an emulsifier, the fat emulsion further comprising a stabilizer selected from the following (a), (b), (c), or (d):
   (a) at least one phospholipid selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid;
   (b) at least one phospholipid derivative selected from phosphatidylethanolamines modified with polyalkyleneglycol, wherein a fatty acid esterified to a glycerol moiety is a C₁₀₋₂₂ linear or branched, saturated or unsaturated fatty acid;
   (c) at least one fatty acid selected from the group consisting of C₁₀₋₂₂ linear or branched, saturated or unsaturated fatty acids; or
   (d) a mixture of at least two members selected from the above groups (a), (b), and (C), wherein
   the stabilizer (a), (b), or (c) is present at a concentration of 0.01 to 1 w/v%, 0.01 to 1 w/v%, 0.05 to 5 w/v%, respectively, per the total amount of fat emulsion and local anaesthetic to be mixed therewith before use, i.e., in a fat emulsion with which a local anaesthetic is mixed before use.
Item 2. The fat emulsion according to Item 1, wherein
   (1) propofol is present at a concentration of 0.4 to 5 w/v%,
   (2) an oily component is present at a concentration of 2 to 20 w/v%, and
   (3) an emulsifier is present at a concentration of 0.4 to 5 w/v%, per the total amount of fat emulsion and local anaesthetic to be mixed therewith before use.
Item 3. The fat emulsion according to Item 1 or 2, wherein a local anaesthetic is at least one member selected from the group consisting of lidocaine, mepivacaine, bupivacaine, ropivacaine, dibucaine, procaine, procaine chloride, tetracaine and pharmacologically acceptable acid addition salts thereof.
Item 4. The fat emulsion according to any one of Items 1 to 3, wherein the local anaesthetic is present at a concentration of 0.01 to 1 w/v%, per the total amount of fat emulsion and local anaesthetic to be mixed therewith before use.
Item 5. The fat emulsion according to any one of Items 1 to 3, wherein the stabilizer is at least one phospholipid (a) selected from the group consisting of phosphatidylglycerol, phosphatidic acid,
   phosphatidylinositol, and phosphatidylserine wherein a fatty acid esterified to a glycerol moiety is a C₁₀₋₂₂ linear or branched, saturated or unsaturated fatty acid.
Item 6. The fat emulsion according to any one of Items 1 to 3, wherein the stabilizer is at least one phospholipid (a) selected from the group consisting of phosphatidylglycerol, phosphatidic acid,
   phosphatidylinositol, and phosphatidylserine wherein a fatty acid esterified to a glycerol moiety is a C₁₂₋₁₈ linear or branched, saturated or unsaturated fatty acid.
Item 7. The fat emulsion according to any one of Items 1 to 3, wherein the stabilizer is at least one phospholipid (a) selected from the group consisting of distearoylphosphatidylglycerol, dipalmitoylphosphatidylglycerol, dioleoylphosphatidylglycerol, distearoylphosphatidic acid, dipalmitoylphosphatidic acid, dioleoylphosphatidic acid, distearoylphosphatidylinositol, dipalmitoylphosphatidylinositol, dioleoylphosphatidylinositol, distearoyphosphatidylserine, dipalmitoylphosphatidylserine, and dioleoylphosphatidylserine.
Item 8. The fat emulsion according to any one of Items 5 to 7, wherein the stabilizer is present at a concentration of 0.03 to 1 w/v%, per the total amount of fat emulsion and local anaesthetic to be mixed therewith before use.
Item 9. The fat emulsion according to any one of Items 1 to 3, wherein the stabilizer is at least one phospholipid derivative (b) selected from phosphatidylethanolamines modified with polyalkyleneglycol, wherein a fatty acid esterified to a glycerol moiety is a C₁₀₋₂₂ linear or branched, saturated or unsaturated fatty acid.
Item 10. The fat emulsion according to any one of Items 1 to 3, wherein the stabilizer is at least one phospholipid derivative (b) selected from phosphatidylethanolamines modified with polyalkyleneglycol having an average molecular weight of 1000 to 5000, wherein a fatty acid esterified to a glycerol moiety is a C₁₄₋₁₈ linear or branched, saturated or unsaturated fatty acid.
Item 11. The fat emulsion according to any one of Items 1 to 3, wherein the stabilizer is at least one phospholipid derivative (b) selected from the group consisting of distearoylphosphatidylethanolamine-polyethylene glycol 5000, distearoylphosphatidylethanolamine-polyethylene glycol 3000, and distearoylphosphatidylethanolamine-polyethylene glycol 2000.
Item 12. The fat emulsion according to any one of Items 9 to 11, wherein the stabilizer is present at a concentration of 0.1 to 1 w/v% when the local anaesthetic is mixed therewith before use.
Item 13. The fat emulsion according to any one of Items 1 to 3, wherein the stabilizer is at least one fatty acid (c) selected from the group consisting of C₁₀₋₂₂ linear or branched, saturated or unsaturated fatty acids.
Item 14. The fat emulsion according to any one of Items 1 to 3, wherein the stabilizer is at least one fatty acid (c) selected from the group consisting of C₁₀₋₂₀ linear or branched, saturated or unsaturated fatty acids.
Item 15. The fat emulsion according to any one of Items 1 to 3, wherein the stabilizer is at least one fatty acid (c) selected from the group consisting of decanoic acid, lauric acid, myristic acid, palmitic acid, stearic acid, arachidic acid, isomyristic acid, isopalmitic acid, and oleic acid.
Item 16. The fat emulsion according to any one of Items 13 to 15, wherein the stabilizer is present at a concentration of 0.1 to 5 w/v%, per the total amount of fat emulsion and local anaesthetic to be mixed therewith before use.
Item 17. A fat-emulsion containing container having at least two compartments (multi-compartment) that is divided with a partition in such a manner as to allow the compartments to communicate with one another, which container comprising one compartment containing the fat emulsion according to any one of Items 1 to 3 and another compartment containing a local anaesthetic.
Item 18. A pain-relieving fat emulsion comprising propofol, an oily component, anemulsifier, a stabilizer, and a local anaesthetic, wherein the stabilizer is selected from the following (a), (b), (c), or (d) :
   (a) at least one phospholipid selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid;
   (b) at least one phospholipid derivative selected from phosphatidylethanolamines modified with polyalkyleneglycol, wherein a fatty acid esterified to a glycerol moiety is a C₁₀₋₂₂ linear or branched, saturated or unsaturated fatty acid;
   (c) at least one fatty acid selected from the group consisting of C₁₀₋₂₂ linear or branched, saturated or unsaturated fatty acids; or
   (d) a mixture of at least two members selected from the above groups (a), (b), and (C), wherein
   the stabilizer (a), (b), or (c) is present at a concentration of 0.01 to 1 w/v%, 0.01 to 1 w/v%, and 0.05 to 5 w/v%, respectively in the fat emulsion.
Item 19. The pain-relieving fat emulsion according to Item 18, wherein
   (1) propofol is present at a concentration of 0.4 to 5 w/v%,
   (2) an oily component is present at a concentration of 2 to 20 w/v%,
   (3) an emulsifier is present at a concentration of 0.4 to 5 w/v%, and
   (4) a local anaesthetic is present at a concentration of 0.01 to 1 w/v%, in the fat emulsion.
Item 20. The pain-relieving fat emulsion according to Item 18 or 19, wherein a local anaesthetic is at least one member selected from the group consisting of lidocaine, mepivacaine, bupivacaine, ropivacaine, dibucaine, procaine, procaine chloride, tetracaine and pharmacologically acceptable acid addition salts thereof.
Item 21. The pain-relieving fat emulsion according to any one of Items 18 to 20, wherein the stabilizer is at least one phospholipid (a) selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid.
Item 22. The pain-relieving fat emulsion according to any one of Items 18 to 20, wherein the stabilizer is at least one phospholipid (a) selected from the group consisting of phosphatidylglycerol, phosphatidic acid, phosphatidylinositol, and phosphatidylserine wherein a fatty acid esterified to a glycerol moiety is a C₁₂₋₁₈ linear or branched, saturated or unsaturated fatty acid.
Item 23. The pain-relieving fat emulsion according to any one of Items 18 to 20, wherein the stabilizer is at least one phospholipid (a) selected from the group consisting of distearoylphosphatidylglycerol, dipalmitoylphosphatidylglycerol, dioleoylphosphatidylglycerol, distearoylphosphatidic acid, dipalmitoylphosphatidic acid, dioleoylphosphatidic acid, distearoylphosphatidylinositol, dipalmitoylphosphatidylinositol, dioleoylphosphatidylinositol, distearoylphosphatidylserine, dipalmitoylphosphatidylserine, and dioleoylphosphatidylserine.
Item 24. The pain-relieving fat emulsion according to any one of Items 21 to 23, wherein the stabilizer is present at a concentration of 0.03 to 1 w/v% in the fat emulsion.
Item 25. The pain-relieving fat emulsion according to any one of Items 18 to 20, wherein the stabilizer is at least one phospholipid derivative (b) selected from phosphatidylethanolamines modified with polyalkyleneglycol, wherein a fatty acid esterified to a glycerol moiety is a C₁₀₋₂₂ linear or branched, saturated or unsaturated fatty acid.
Item 26. The pain-relieving fat emulsion according to any one of Items 18 to 20, wherein the stabilizer is at least one phospholipid derivative (b) selected from phosphatidylethanolamines modified with polyalkyleneglycol having the average molecular weight of 1000 to 5000, wherein a fatty acid esterified to a glycerol moiety is a C₁₄₋₁₈ linear or branched, saturated or unsaturated fatty acid.
Item 27. The pain-relieving fat emulsion according to any one of Items 18 to 20, wherein the stabilizer is at least one phospholipid derivative (b) selected from the group consisting of distearoylphosphatidylethanolamine-polyethylene glycol 5000, distearoylphosphatidylethanolamine-polyethylene glycol 3000, and distearoylphosphatidylethanolamine-polyethylene glycol 2000.
Item 28. The pain-relieving fat emulsion according to any one of Items 25 to 27, wherein the stabilizer is present at a concentration of 0. 1 to 1 w/v% in the fat emulsion.
Item 29. The pain-relieving fat emulsion according to any one of Items 18 to 20, wherein the stabilizer is at least one fatty acid (c) selected from the group consisting of C₁₀₋₂₂ linear or branched, saturated or unsaturated fatty acids.
Item 30. The pain-relieving fat emulsion according to any one of Items 18 to 20, wherein the stabilizer is at least one fatty acid (c) selected from the group consisting of C₁₀₋₂₀ linear or branched, saturated or unsaturated fatty acids.
Item 31. The pain-relieving fat emulsion according to any one of Items 18 to 20, wherein the stabilizer is at least one fatty acid (c) selected from the group consisting of oleic acid, isomyristic acid, isopalmitic acid, decanoic acid, lauric acid, myristic acid, palmitic acid, stearic acid, and arachidic acid.
Item 32. The pain-relieving fat emulsion according to any one of Items 29 to 31, wherein the stabilizer is present at a concentration of 0.05 to 0.2 w/v% in the fat emulsion.
Item 33. A method for manufacturing a pain-relieving fat emulsion, the method comprising:
   mixing a local anaesthetic with a fat emulsion comprising propofol, an oily component, an emulsifier, and a stabilizer, wherein the stabilizer is selected from the following (a), (b), (c), or (d) :
      (a) at least one phospholipid selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid;
      (b) at least one phospholipid derivative selected from phosphatidylethanolamines modified with polyalkyleneglycol, wherein a fatty acid esterified to a glycerol moiety is a C₁₀₋₂₂ linear or branched, saturated or unsaturated fatty acid;
      (c) at least one fatty acid selected from the group consisting of C₁₀₋₂₂ linear or branched, saturated or unsaturated fatty acids; or
      (d) a mixture of at least two members selected from the above groups (a), (b), and (c),
   to thereby obtain a fat emulsion wherein
      the stabilizer (a), (b), or (c) is present at a concentration of 0.01 to 1 w/v%, 0.01 to 1 w/v%, and 0.05 to 5 w/v%, respectively, in the fat emulsion.
Item 34. Use of the following stabilizers (a) to (d) for stabilizing a fat emulsion with which a local anaesthetic is mixed before use and which comprises propofol, an oily component, and an emulsifier, or a pain-relieving fat emulsion which comprises propofol, an oily component, an emulsifier, and a local anaesthetic:
   (a) at least one phospholipid selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid;
   (b) at least one phospholipid derivative selected from phosphatidylethanolamines modified with polyalkyleneglycol, wherein a fatty acid esterified to a glycerol moiety is a C₁₀₋₂₂ linear or branched, saturated or unsaturated fatty acid;
   (c) at least one fatty acid selected from the group consisting of C₁₀₋₂₂ linear or branched, saturated or unsaturated fatty acids; or
   (d) a mixture of at least two members selected from the above groups (a), (b), and (c).

In particular, the present invention provides the inventions of Items [1] to [27]:
[1] A propofol-containing fat emulsion with which a local anaesthetic is mixed before use for relieving pain, the propofol-containing fat emulsion comprising as a stabilizer:
   0.01 to 1 wt% of at least one phospholipid selected from the group consisting of phosphatidylglycerol, phosphatidic acid, phosphatidylinositol, and phosphatidylserine wherein a linear or branched, saturated or unsaturated fatty acid with a C₁₀₋₂₂ main chain is a constituent fatty acid component; and/or
   0.2 to 5 wt% of at least one linear or branched, saturated or unsaturated fatty acid with a C₁₀₋₂₂ main chain.
[2] The propofol-containing fat emulsion according to Item [1], which comprises the following components (2) to (5) in such a manner as to yield the following final concentrations after a local anaesthetic (1) is mixed with the fat emulsion before use:
   (1) 0.01 to 1 wt% of at least one local anaesthetic for relieving pain;
   (2) 0.01 to 1 wt% of at least one phospholipid selected from the group consisting of phosphatidylglycerol, phosphatidic acid,
      phosphatidylinositol, and phosphatidylserine wherein a linear or branched, saturated or unsaturated fatty acid with a C₁₀₋₂₂ main chain is a constituent fatty acid component; and/or 0.2 to 5 wt% of at least one linear or branched, saturated or unsaturated fatty acid with a C₁₀₋₂₂ main chain;
   (3) 0.5 to 5 wt% of propofol;
   (4) 2 to 20 wt% of an oily component; and
   (5) 0.5 to 5 wt% of an emulsifier.
[3] The fat emulsion according to Item [1], wherein the local anaesthetic is at least one member selected from the group consisting of lidocaine, mepivacaine, bupivacaine, ropivacaine, dibucaine, procaine, procaine chloride, tetracaine and pharmacologically acceptable acid addition salts thereof.
[4] The propofol-containing fat emulsion according to Item [3], wherein the local anaesthetic is at least one member selected from the group consisting of lidocaine and its hydrochloride.
[5] The propofol-containing fat emulsion according to Item [1], wherein the local anaesthetic is mixed therewith before use in such a manner as to yield a final concentration of 0.01 to 1 wt%.
[6] The propofol-containing fat emulsion according to Item [1], wherein the stabilizer is at least one phospholipid selected from the group consisting of phosphatidylglycerol, phosphatidic acid,
   phosphatidylinositol, and phosphatidylserine wherein a linear or branched, saturated or unsaturated fatty acid with a C₁₀₋₂₂ main chain is a constituent fatty acid component.
[7] The propofol-containing fat emulsion according to Item [6], wherein the stabilizer is a phospholipid selected from the group consisting of distearoylphosphatidylglycerol, distearoylphosphatidic acid, distearoylphosphatidylinositol, and distearoylphosphatidylserine.
[8] The propofol-containing fat emulsion according to Item [7], wherein the stabilizer is a phospholipid selected from the group consisting of distearoylphosphatidylglycerol and distearoylphosphatidic acid.
[9] The propofol-containing fat emulsion according to any one of Items [6] to [8], wherein the content of the stabilizer is 0.01 to 0.7 wt% in a mixture obtained by mixing a local anaesthetic with the fat emulsion before use in such a manner as to yield a final concentration of the local anaesthetic of 0.01 to 1 wt%.
[10] The propofol-containing fat emulsion according to Item [1], wherein a stabilizer is a linear or branched, saturated or unsaturated fatty acid with a C₁₀₋₂₂ main chain.
[11] The propofol-containing fat emulsion according to Item [10], wherein the stabilizer is a fatty acid selected from the group consisting of oleic acid, myristic acid, palmitic acid, and stearic acid.
[12] The propofol-containing fat emulsion according to Item [11], wherein the stabilizer is oleic acid.
[13] The propofol-containing fat emulsion according to any one of Items [10] to [12], wherein the content of the stabilizer is 0.2 to 2 wt% in a mixture obtained by mixing a local anaesthetic with the fat emulsion before use in such a manner as to have a final concentration of the local anaesthetic of 0.01 to 1 wt%.
[14] A fat-emulsion containing container, comprising a flexible container having a multi-compartment that is divided with a partition in such a manner as to allow the compartments to communicate with one another, wherein one compartment of the multi-compartment contains the fat emulsion according to Item [1] and another compartment contains a local anaesthetic.
[15] A pain-relieving propofol-containing fat emulsion comprising:
   0.01 to 1 wt% of a local anaesthetic for relieving pain; and
   as a stabilizer, 0.01 to 1 wt% of at least one phospholipid selected from the group consisting of phosphatidylglycerol, phosphatidic acid, phosphatidylinositol, and phosphatidylserine wherein a linear or branched, saturated or unsaturated fatty acid with a C₁₀₋₂₂ main chain is a constituent fatty acid component; and/or 0.2 to 5 wt% of at least one linear or branched, saturated or unsaturated fatty acid with a C₁₀₋₂₂ main chain.
[16] The propofol-containing fat emulsion according to Item [15], comprising the following components in such a manner as to yield the following final concentrations:
   (1) 0.01 to 1 wt% of at least one local anaesthetic for relieving pain;
   (2) 0.01 to 1 wt% of at least one phospholipid selected from the group consisting of phosphatidylglycerol, phosphatidic acid,
      phosphatidylinositol, and phosphatidylserine wherein a linear or branched, saturated or unsaturated fatty acid with a C₁₀₋₂₂ main chain is a constituent fatty acid component; and/or 0.2 to 5 wt% of at least one linear or branched, saturated or unsaturated fatty acid with a C₁₀₋₂₂ main chain;
   (3) 0.5 to 5 wt% of propofol;
   (4) 2 to 20 wt% of an oily component; and
   (5) 0.5 to 5 wt% of an emulsifier.
[17] The propofol-containing fat emulsion according to Item [15], wherein the local anaesthetic is at least one member selected from the group consisting of lidocaine, mepivacaine, bupivacaine, ropivacaine, dibucaine, procaine, procaine chloride, tetracaine and pharmacologically acceptable acid addition salts thereof.
[18] The propofol-containing fat emulsion according to Item [17], wherein the local anaesthetic is at least one member selected from the group consisting of lidocaine and its hydrochloride.
[19] The propofol-containing fat emulsion according to Item [15], wherein the stabilizer is at least one phospholipid selected from the group consisting of phosphatidylglycerol, phosphatidic acid, phosphatidylinositol, and phosphatidylserine wherein a linear or branched, saturated or unsaturated fatty acid with a C₁₀₋₂₂ main chain is a constituent fatty acid component.
[20] The propofol-containing fat emulsion according to Item [19], wherein the stabilizer is a phospholipid selected from the group consisting of distearoylphosphatidylglycerol, distearoylphosphatidic acid, distearoylphosphatidylinositol, and distearoylphosphatidylserine.
[21] The propofol-containing fat emulsion according to Item [20], wherein the stabilizer is a phospholipid selected from the group consisting of distearoylphosphatidylglycerol and distearoylphosphatidic acid.
[22] The propofol-containing fat emulsion according to any one of Items [19] to [21], wherein the content of the stabilizer is 0.01 to 0.7 wt%.
[23] The propofol-containing fat emulsion according to Item [15], wherein the stabilizer is a linear or branched, saturated or unsaturated fatty acid with a C₁₀₋₂₂ main chain.
[24] The fat emulsion according to Item [23], wherein the stabilizer is a fatty acid selected from the group consisting of oleic acid, myristic acid, palmitic acid, and stearic acid.
[25] The fat emulsion according to Item [24], wherein the stabilizer is oleic acid.
[26] The propofol-containing fat emulsion according to any one of Items [23] to [25], wherein the content of the stabilizer is 0.2 to 2 wt%.
[27] A method for manufacturing a pain-relieving propofol-containing fat emulsion, the method comprising mixing a local anaesthetic for relieving pain with a propofol-containing fat emulsion before use, the propofol-containing fat emulsion comprising as a stabilizer:
   0.01 to 1 wt% of at least one phospholipid selected from the group consisting of phosphatidylglycerol, phosphatidic acid, phosphatidylinositol, and phosphatidylserine wherein a linear or branched, saturated or unsaturated fatty acid with a C₁₀₋₂₂ main chain is a constituent fatty acid component; and/or
   0.2 to 5 wt% of at least one linear or branched, saturated or unsaturated fatty acid with a C₁₀₋₂₂ main chain.

The fat emulsion of the present invention is described below in more detail.

### 1. Fat emulsion with which a local anaesthetic is mixed before use

The fat emulsion of the invention with which a local anaesthetic is mixed before use comprises propofol, which is generally known as an anesthetic or sedative (hereinafter sometimes referred to as "fat emulsion of the invention"). The fat emulsion of the invention comprises propofol, an oily component, and an emulsifier, and further comprises a specific stabilizer in a prescribed amount.

### (1-1) Local anaesthetic

Various local anaesthetics known for the effect of relieving pain can be mixed with the fat emulsion of the invention before use. Such local anaesthetics include lidocaine, mepivacaine, bupivacaine, ropivacaine, dibucaine, procaine, procaine chloride, tetracaine and pharmacologically acceptable acid addition salts thereof. These local anaesthetics may be used singly or in combination.

The local anaesthetic may be mixed with the fat emulsion of the invention before use in an amount sufficient to prevent or alleviate pain (vascular pain) occurring during the administration thereof by intravenous injection or infusion. The amount of the local anaesthetic sufficient to relieve pain is favorably determined in such a manner as to yield a concentration of about 0.01 to about 1 w/v%, and preferably about 0.01 to about 0.5 w/v% in the fat emulsion prepared by mixing it with the local anaesthetic. Hereinafter, this concentration is sometimes referred to as "final concentration".

In this specification, the concentration of the above-described local anaesthetic and each component forming the fat emulsion of the invention is represented by "w/v%". This "w/v%" refers to [the weight of the component (g)]/[the volume of the fat emulsion (100 ml)].

### (1-2) Fat emulsion

In the invention, a fat emulsion with which a stabilizer is mixed is prepared by emulsifying propofol as an active component and an oily component (lipid component) into water using a suitable emulsifier. Typical examples of the fat emulsion are commercially available propofol fat emulsions for intravenous administration.

In general, vegetable oils are employed as the oily component for preparing the fat emulsions. Examples of such vegetable oils include soybean oil, cotton seed oil, rapeseed oil, sesame oil, corn oil, peanut oil, safflower oil, olive oil, castor oil, etc. The oily component may be a medium chain triglyceride. Specific examples of the usable oily components include various commercially available products, such as COCONARD (registered trademark, manufactured by Kao Corporation), ODO (registered trademark, manufactured by Nisshin Oil Mills, Ltd.), Miglyol (registered trademark, manufactured by SASOL Ltd.), Panasate (registered trademark, manufactured by NOF Corporation), etc. These vegetable oils and medium chain triglycerides can be used singly or in combination of two or more members suitably selected from the same group (vegetable oils or medium chain triglycerides) or different groups.

The oily components are not limited to the above-described vegetable oils and medium chain triglycerides, and for example, can include animal oils, mineral oils, synthetic oils, and essential oils. These oily components are used singly or in combination. Alternatively, animal oils may be combined with vegetable oils and/or medium chain triglycerides.

Typical examples of emulsifiers include natural phospholipids, such as egg yolk lecithin and soybean lecithin, and hydrogenated egg yolk lecithin and hydrogenated soybean lecithin, etc. Chemically synthesized phosphatidylcholines and phosphatidylethanolamines may be also used as an emulsifier.

Examples of chemically synthesized phosphatidylcholines include dipalmitoylphosphatidylcholine, dimyristoylphosphatidylcholine, distearoylphosphatidylcholine, dioleoylphosphatidylcholine, etc. Examples of chemically synthesized phosphatidylethanolamines include dipalmitoylphosphatidylethanolamine, dimyristoylphosphatidylethanolamine, distearoylphosphatidylethanolamine, dioleoylphosphatidylethanolamine, etc.

These emulsifiers can be used singly or in combination. Among the above, egg yolk lecithin and soybean lecithin are preferable as an emulsifier.

Processes for emulsifying the propofol as an active component and an oily component using an emulsifier are well known by persons skilled in the art. Examples of the processes include a process comprising the steps of adding water for injection to a mixture of an active component, an oily component, and an emulsifier to form a rough emulsion, and subsequently using a suitable high-pressure emulsifying device, etc., to refine the rough emulsion.

Each proportion of the active component, oily component, and emulsifier is suitably selected from the following ranges: 0.4 to 5 w/v% of propofol, 2 to 20 w/v% of the oily component and 0.4 to 5 w/v% of the emulsifier in terms of the concentration of the finished product after a local anaesthetic is mixed with the emulsified liquid (fat emulsion) that is obtained. The concentration of the finished product is represented by a percentage of weight to volume thereof (final concentration, w/v%). A particularly preferable proportion thereof is selected from the following ranges: 0.5 to 2 w/v% of propofol, 5 to 10 w/v% of the oily component, and 0.5 to 2 w/v% of the emulsifier in terms of the final concentration.

To the emulsified liquid prepared above may be further added various additives in a suitable amount as required, but this is not absolutely necessary. Examples of the additives include antioxidants, antibacterial agents, pH adjusting agents, isotonizing agents, etc., which are known for their mixing capability with this type of emulsified liquid. Specific examples of the antioxidants include sodium metabisulfite (which serves as an antibacterial agent and an antioxidant), sodium sulfite, sodium bisulfite, potassium metabisulfite, potassium sulfite, etc. Examples of the antibacterial agents include sodium caprylate,methyl benzoate,sodiummetabisulfite, sodium edetate, etc. Sodium hydroxide, hydrochloric acid, etc., can be used as a pH adjusting agent. Examples of the isotonizing agents include glycerols; saccharides such as glucose, fructose, maltose, etc.; and sugar alcohols, such as sorbitol, xylitol, etc. Among these, oil soluble additives can be mixed beforehand with an oily component of an emulsified liquid, and water-soluble additives can be mixed with water for injection or admixed to the aqueous phase of the emulsified liquid obtained. The amount of each additive is obvious for persons skilled in the art, and is not noticeably different from conventionally-employed amounts.

### (1-3) Stabilizer

A stabilizer in the invention is at least one member selected from the group consisting of the following (a) to (d).
(a) at least one phospholipid selected from the group consisting of phosphatidylglycerol, phosphatidic acid, phosphatidylinositol, and phosphatidylserine wherein a fatty acid esterified to a glycerol moiety is a C₁₀₋₂₂, preferably a C₁₂₋₁₈, linear or branched, saturated or unsaturated fatty acid;
(b) at least one phospholipid derivative selected from phosphatidylethanolamines modified with polyalkyleneglycol, wherein a fatty acid esterified to a glycerol moiety is a C₁₀₋₂₂, preferably a C₁₄₋₁₈, linear or branched, saturated or unsaturated fatty acid;
(c) at least one fatty acid selected from the group consisting of C₁₀₋₂₂, preferably C₁₀₋₂₀, linear or branched, saturated or unsaturated fatty acids; or
(d) a mixture of at least two members selected from the above groups (a), (b), and (c) .

In the above Items (a) and (b), "glycerol moiety" denotes the portions shown in the following formulae which illustrate the structure of phosphatidylglycerol, phosphatidic acid, phosphatidylinositol, phosphatidylserine, and phosphatidylethanolamine wherein a constituent fatty acid is a palmitic acid.

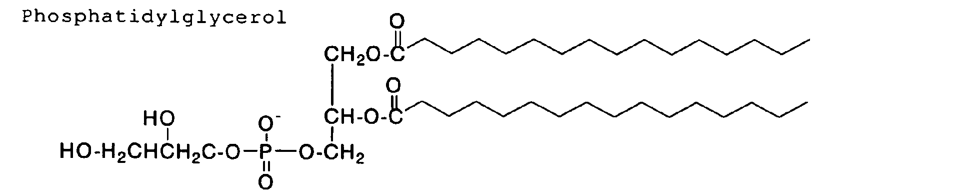

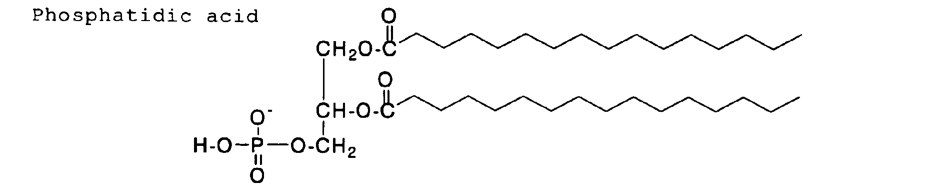

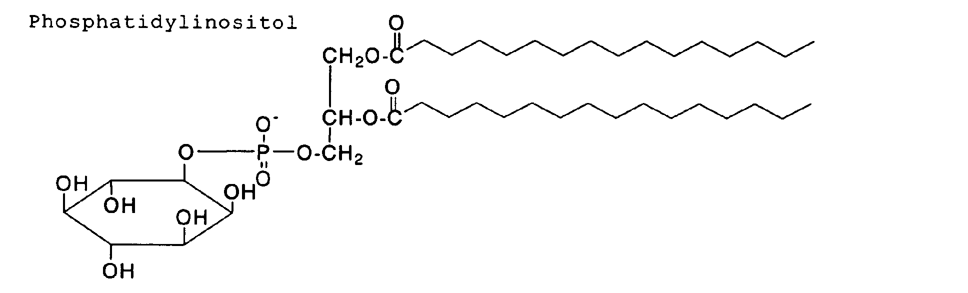

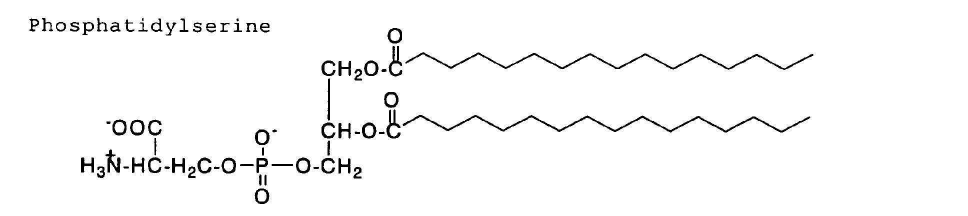

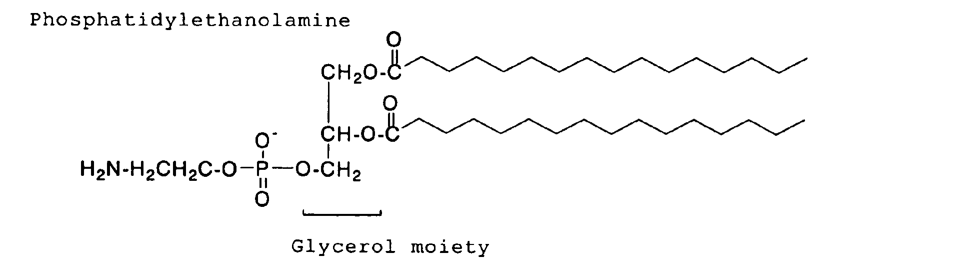

A linear or branched, saturated or unsaturated fatty acid which exists in nature can be mentioned as an example of a fatty acid forming a phospholipid (a) (i.e., a fatty acid esterified to a glycerol moiety of phospholipid (a)), a fatty acid forming a phospholipid derivative (b) (i.e., a fatty acid esterified to a glycerol moiety of a phospholipid derivative (b)), and a fatty acid (c). The above-described fatty acids include linear fatty acids, such as capric acid, lauric acid, myristic acid, palmitic acid, stearic acid, arachidic acid, behenic acid, oleic acid, linoleic acid, linolenic acid, arachidonic acid, and eicosapentaenoic acid; and branched chain fatty acids such as isomyristic acid, isopalmitic acid, isostearic acid, isoarachidonic acid, etc.

Specific examples of phospholipids (a) comprising the above-described fatty acid as a constituent fatty acid component include dicaproylphosphatidylglycerol, dilauroylphosphatidic acid, dimyristoylphosphatidylinositol, dipalmitoylphosphatidylserine, distearoylphosphatidylglycerol, diarachidoylphosphatidylglycerol, dibehenylphosphatidic acid, distearoylphosphatidylinositol, distearoylphosphatidic acid, distearoylphosphatidylserine, diisomyristoylphosphatidylglycerol, diisostearoylphosphatidic acid, diisoarachidoylphosphatidylinositol, etc.

Among these, distearoylphosphatidylglycerol, distearoylphosphatidic acid, distearoylphosphatidylinositol, and distearoylphosphatidylserine are preferable, and distearoylphosphatidylglycerol and distearoylphosphatidic acid are more preferable.

The phospholipid derivatives (b) comprising the above-described fatty acid as a constituent fatty acid component include phosphatidylethanolamines modified with polyalkyleneglycol represented by General Formula (I) 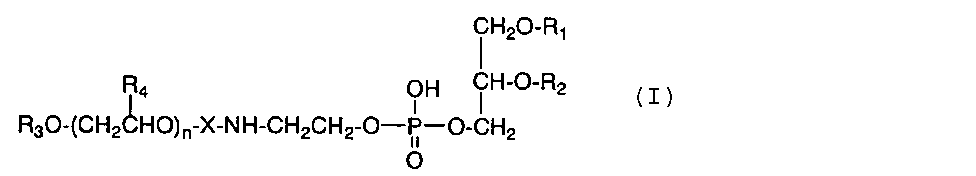 wherein R₁ and R₂ each represent a fatty acid residue (constituent fatty acid component), R₃ and R₄ each represent a hydrogen atom or a methyl group, X represents a group of -CO(CH₂)₂CO-, -CO(CH₂)₃CO-, or CO-, and n is an integer of 20 to 120.

More specifically, as shown in General Formula (I), the polyalkyleneglycol modifying phosphatidylethanolamine in the phospholipid derivatives (b) is bonded to an amino group of phosphatidylethanolamine via an X group as a substituent (substituent at a nitrogen atom). The polyalkylene glycol is polyethylene glycol or polypropylene glycol.

In the invention, preferable examples of phospholipid derivative (b) are distearoylphosphatidylethanolamine-polyethylene glycol 5000 (trade name "SUNBRIGHT DSPE-050C", manufactured by NOF CORPORATION), distearoylphosphatidylethanolamine-polyethylene glycol 3000 (trade name "MPEG 3000PE", manufactured by Avanti Polar Lipids, Inc.), and distearoylphosphatidylethanolamine-polyethylene glycol 2000 (trade name "SUNBRIGHT DSPE-020CN", manufactured by NOF CORPORATION) .

The value shown in each of the above-mentioned compound names is the average molecular weight of polyethylene glycol. The average molecular weight of polyalkylene glycol, such as the above-mentioned polyethyleneglycol, etc., is preferably within the range of 1000 to 5000.

The compounds belonging to the above-mentioned each group of phospholipid (a), phospholipid derivative (b), or fatty acid (c) can be used singly, or in combination. Alternatively, the invention, allows the combined use of different types of compounds suitably selected from the above-mentioned groups of phospholipid (a), phospholipid derivative (b), and fatty acid (c).

The phospholipid (a) to be mixed with the fat emulsion is preferably present at a final concentration of 0.01 to 1 w/v%, more preferably 0.03 to 1 w/v% based on the total amount of a product prepared by mixing a local anaesthetic with the obtained fat emulsion of the invention before use. When two or more phospholipids are combined, the above range applies to the total amount thereof, and hereinafter the same shall apply. By using the phospholipid within the above range, the effect of retaining excellent emulsion stability can be exhibited as intended by the invention when a local anaesthetic is mixed with the fat emulsion of the invention before use.

The phospholipid derivative (b) to be mixed with the fat emulsion is preferably present at a final concentration of 0.01 to 1 w/v%, more preferably 0.1 to 1 w/v% based on the total amount of a product prepared by mixing the local anesthetic with the obtained fat emulsion of the invention before use. When two or more of phospholipid derivatives are combined, the above range applies to the total amount thereof, and hereinafter the same shall apply. By using the phospholipid derivative (s) within the above range, the effect of retaining the excellent emulsion stability can be exhibited as intended by the invention when a local anaesthetic is mixed with the obtained fat emulsion of the invention before use.

A suitable amount of the fatty acid (c) to be mixed with the fat emulsion is as follows: 0.05 to 5 w/v% at a final concentration based on the total amount of a product prepared by mixing the local anaesthetic with the fat emulsion of the invention before use; 0.1 to 5 w/v% at a final concentration, in the case of a kit preparation under Item (2) described later; 0.05 to 0.2 w/v% in the case of a pain-relieving fat emulsion formulation (premix formulation) comprising a local anaesthetic under Item (3) described later. When two or more fatty acids are combined, the above range applies to the total amount thereof. By the use of the fatty acid(s) within the above range, the effect of retaining the excellent emulsion stability intended by the invention can be exhibited when a local anaesthetic is mixed with the fat emulsion of the invention before use.

In the case of the combined use of two or more members selected from different groups of the phospholipid (a), the phospholipid derivative (b), and the fatty acid (c), it is advantageous that the amount of each component is suitably selected from the above range. However, the amount of each component may be lower than the lower limit of the above range because a synergistic effect can be expected from the combined use. In general, the total amount of the components to be combined canbe selected from the range of 0.01 to 1 w/v%, and preferably 0.01 to 0. 7 w/v%. The effect intended by the invention can be achieved by combined use of these components even in a reduced amount.

### (1-4) Preparation of the fat emulsion of the invention

The stabilizers mentioned in Item (1-3) above are further added to a mixture of an active component, an oily component, and an emulsifier, and, as required, an additive (an oil-soluble additive), before the emulsification process for obtaining the fat emulsion described in Item (1-2) above. The stabilizer-containing mixture thus obtained is emulsified in water for injection, etc., to produce the desired fat emulsion of the invention. This emulsification can be carried out in the same manner as in Item (1-2) above. Examples of processes include a process comprising the steps of adding water for injection and, as desired, a water-soluble additive to a mixture of an active component, an oily component, and an emulsifier, and, as required, an oil-soluble additive, to form a rough emulsion, and subsequently using an appropriate high-pressure emulsifying device, etc., to refine the rough emulsion.

The above-described emulsification does not need to be conducted under normal temperatures, and can be carried out under suitable heating conditions (e.g., 80°C or lower, and preferably about 40°C to about 70°C), which are commonly known in the art, such as in ordinary emulsification processes of fat emulsions.

### (1-5) Fat emulsion product of the invention

To the fat emulsion of the invention thus prepared is further added, as required, a suitable pH adjusting agent so as to adjust the pH within the range of 8.2 to 8.3 depending on the type of each component contained therein, however the addition of the pH adjusting agent is not absolutely necessary. The obtained fat emulsion is placed in a suitable container, such as a vial, syringe, plastic bag, ampule, etc., according to common procedures, and is then sterilized using a commonly-used sterilization process, preferably high-pressure steam sterilization using an autoclave (for example, 121°C, 12 minutes), for commercial production.

The obtained product is given excellent emulsion stability by the addition of the specific stabilizer thereto. Needless to say, the stabilizer itself has excellent safety, and thus the obtained product is characterized by high safety. In particular, the emulsion stability is not substantially impaired even when a local anaesthetic, such as lidocaine, is mixed with the fat emulsion before use in an amount sufficient to remove pain. Further, the above-described emulsion stability is not lost even when a heat sterilization process, such as high-pressure steam sterilization, etc., is conducted, and thereby maintains the above-described favorable emulsion state. The above-described emulsion stability is maintained even after extended storage (e.g., for 6 months at 40°C).

In order to obtain these effects, the suitable proportion and more preferable proportion (final concentration) of each component forming the fat emulsion of the invention are as shown in Table 1-A below.

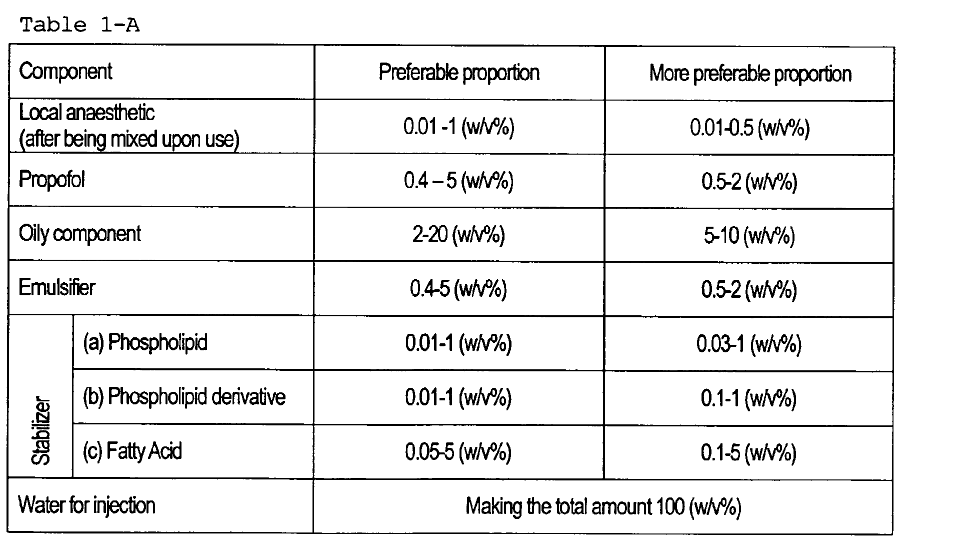

In addition to the excellent emulsion stability described above, the fat emulsion of the invention has an advantage in that the mean particle diameter of the emulsion particles is as fine as about 0.3 µm or less, and the particle size does not substantially change before and after sterilization. Further, the fat emulsion of the invention is stable within in the pH range of about 5 to 9, which is suitable for injection; temperature stability thereof is excellent; and it withstands extended storage because emulsion particles are negatively charged (no degradation after six months' storage at 40°C) . The fat emulsion of the invention can be prepared in such a manner as to have the same viscosity and osmotic pressure as commercially available nutrition fat emulsions, and such a fat emulsion can ease the patient's discomfort during the administration thereof. The propofol that is contained in the fat emulsion is substantially retained in an oil phase even after mixing the local anaesthetic with the fat emulsion before use.

The fat emulsion of the invention is mixed (e.g., by irrigation) with the above-described local anaesthetic before use and is then administered by intravenous injection or infusion as a general anesthetic or sedative. During the administration thereof, since the emulsified liquid itself is stable, there is no possibility that it may separate into two phases or that emulsion particles may agglomerate. The fat emulsion of the invention is thereby safe to use.

### (1-6) Formulation of the fat emulsion of the invention

The fat emulsion of the invention can be prepared into various formulations suitable for mixing a local anaesthetic therewith before use, such as a kit preparation for injection. Specific examples of such kit preparations include a double bag type, a two-compartment syringe type, an integrated type (a double tipped needle, a combined needle), etc. The above-mentioned kit preparations for injection can be prepared quickly and accurately. In practical use, the prepared formulations can make it possible to avoid risks such as microbial-contamination at the time of mixing the local anaesthetic, needlestick accidents, etc. The agglomeration of emulsion particles can also be avoided in the currently-conducted method of administration using a three way stopcock.

### 2. Kit preparations

The invention also provides a fat emulsion container product (kit preparation) that holds the two above-described agents, i.e., the fat emulsion of the invention and the local anaesthetic, in a specific container.

The fat emulsion container product (kit preparation) of the invention comprises a container holding a fat emulsion. The container has at least two compartments (multi-compartment) which are divided with a partition that also allows the compartments to communicate with each other. One compartment holds the fat emulsion of the invention prepared according to the procedure described in Item (1-4) above and another compartment holds a local anaesthetic.

Specific examples of containers available for the product include a multi-compartment container (at least two compartments) in which the compartments are divided with a divider (partition), such that the compartments can be communicated with each other by opening the partition upon use. Preferably, a flexible container can be given as an example. A double bag as is known in the transfusion field for use with a hemodialysis fluid, etc. can be given as an example of such containers.

The double bag (plastic bag) can be formed from various types of flexible plastics commonly used for medical containers, etc. The plastics preferably have a gas barrier property. Specific examples of such plastics with a gas barrier property include resins, such as polyethylene terephthalate, polyethylene naphthalate, ethylene/vinyl alcohol copolymer, polyvinylidene chloride, polyacrylonitrile, polyvinyl alcohol, polyamide, polyester, etc. A container usable for the invention is suitably formed from a resin film or sheet made of one of the above-mentioned resins, or a film or sheet obtained by evaporating silica, alumina or the like onto one of the above films or sheets, or a laminate of one of the films or sheets.

Specific examples of processes for forming a multi-compartment in the container include (1) a process for forming a partition using an easily peelable welding (Japanese Unexamined Patent Publication No. 1990-4671 and Japanese Unexamined Utility Model Publication No. 1993-5138); (2) a process for forming a partition by clipping compartments (Japanese Unexamined Patent Publication No. 1988-309263); (3) a process for providing various sealable communicating means at the partition (Japanese Examined Patent Publication No. 1988-20550), etc. Among the above, the container of Item (1) is preferable because it is suitable for mass production, and also the compartments can be easily allowed to communicate. More specifically, such a partition can be easily formed in the container by fusion-bonding a part of the inner surface of a plastic bag by using a heat seal bar, etc. The heat-sealed part can be easily opened by low pressure.

The container of the invention holds the stabilized fat emulsion in one compartment of the double bag configured as described above, and holds a local anaesthetic in another compartment thereof. These agents can be placed in the compartments via a member for charging/discharging the agents provided in the upper and lower ends of the container. The product thus obtained can be subjected to high-pressure steam sterilization using an autoclave or the like according to a common process. The product allows each compartment to communicate with each other by opening the partition upon use, whereby the agents held in each compartment are mixed, yielding the desired fat emulsion which produces no pain when administered. The mixed agent can be administered as a general anesthetic or a sedative by intravenous administration or infusion via the agent-discharging member.

### 3. Pain-relieving fat emulsion

The invention provides a fat emulsion which produces no pain when administered, which is obtained by mixing a predetermined amount of local anaesthetic with the fat emulsion of the invention (hereinafter sometimes referred to as "pain-relieving fat emulsion of the invention").

As described above, the inventors found that the fat emulsion obtained by employing a specific stabilizer in a predetermined amount exhibits emulsion stability even when a local anaesthetic for removing pain is mixed with the fat emulsion, and that the stability is not substantially deteriorated after extended storage. The pain-relieving fat emulsion of the invention has been accomplished based on these findings, and is obtained by, in advance, adding a local anaesthetic in an amount sufficient to prevent pain to the fat emulsion of the invention.

The types of local anaesthetics usable in the pain-relieving fat emulsion of the invention may be the same as those to be mixed before use with the fat emulsion of the invention described in the section of "Fat emulsion of the invention". The amount of local anaesthetic to be added to form the pain-relieving fat emulsion is the same as that added to the fat emulsion of the invention before use described above. More specifically, the amount of local anaesthetic to bemixed with the fat emulsion of the invention before use is determined based on the total volume (w/v%) of the fat emulsion with which the local anaesthetic is mixed before use (i.e., pain-relieving fat emulsion comprising the fat emulsion and the local anaesthetic). Thus, the proportion of local anaesthetic in the pain-relieving fat emulsion of the invention as taught in this section can be suitably selected from the range of the above-mentioned amount of local anaesthetic to be mixed before use.

The preferable proportion and more preferable proportion (final concentration) of each component in the pain-relieving fat emulsion of the invention are shown in Table 1-B.

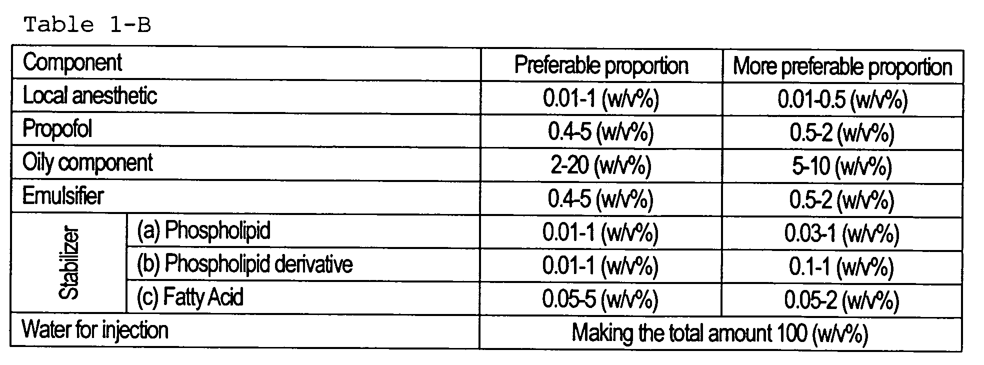

The addition of the local anaesthetic may be carried out either before or after the emulsification process for obtaining the fat emulsion of Item (1-4) above.

The local anaesthetic is mixed before the emulsification process by, for example, mixing propofol, an oily component, an emulsifier, and, as required, an additive (oil-soluble additive), and a local anaesthetic , and emulsifying the obtained mixture in water for injection (towhichawater-solubleadditivecanbeadded, if needed), thereby providing the desired pain-relieving fat emulsion of the invention. This emulsification process can be performed by forming a rough emulsion in advance, and subsequently using a suitable high-pressure emulsifying device, etc., to refine the rough emulsion as described in Item (1-4) above.

The addition of the local anaesthetic after the emulsification process is carried out by adding and mixing the local anaesthetic in a predetermined amount with the fat emulsion of the invention after the emulsification process. More specifically, this process is carried out by, for example, mixing propofol, an oily component, and an emulsifier, and, as required, an additive (oil-soluble additive), emulsifying the obtained mixture in water for injection (to which a water-soluble additive can be added, if needed), and dissolving and mixing the local anaesthetic with the obtained emulsified liquid. Thus, the desired pain-relieving fat emulsion of the invention can be prepared. This emulsification process can be performed by forming a rough emulsion in advance, and subsequently using a suitable high-pressure emulsifying device, etc., to refine the rough emulsion as described in Item (1-4). The emulsion particle size of the emulsified preparation obtained by mixing and dissolving the local anaesthetic therewith after the emulsification process is not substantially different from and is as fine and uniform as that of the emulsified preparation obtained by mixing the local anaesthetic therewith before the emulsification process.

The pain-relieving fat emulsion of the invention thus prepared is placed in a suitable container, such as a vial, syringe, plastic bag, ampule, etc., according to a commonly known procedure, and then is sterilized using a commonly-used sterilization process, preferably a high-pressure steam sterilization using an autoclave (for example, 121°C, 12 minutes), for commercial production.

The product eliminates the necessity for mixing the local anaesthetic with the fat emulsion before use and is easy to handle because a stabilizer with high safety and a local anaesthetic are incorporated beforehand into a single preparation. Moreover, the product of the invention also has the advantage that there is no possibility of medical accidents occurring, such as when the agents in the compartments are not sufficiently mixed before use due to a failure in communication between the compartments, which might occur in the kit preparation. It is more important that the product is characterized by its excellent emulsion stability upon preparation, and the fact that the emulsion stability is maintained without being substantially impaired over an extended period of time.

The product has the following characteristics in addition to excellent emulsion stability: the emulsion particles are negatively charged; the mean particle size thereof is as fine as about 0.3 µm or less; the particle size does not substantially change between the states before and after sterilization; the stability is maintained for an extended period of time within the pH range of about 5 to about 9, which range is suitable for injection; and the temperature stability is excellent (no substantial change in the emulsion particle size after 6 months' storage at 40°C). The pain-relieving fat emulsion of the invention can be prepared in such a manner as to yield the same viscosity and osmotic pressure as in commercially available nutrition fat emulsions, and can ease a patient' s discomfort during the administration thereof. Propofol contained in the fat emulsion is substantially retained stably in the oil phase.

The pain-relieving fat emulsion of the invention can be administered by intravenous injection or infusion as a general anesthetic or sedative in the same manner as in prior-art propofol-containing fat emulsions, and, in particular, has the advantage that pain (vascular pain) does not substantially arise during the administration thereof.

### BEST MODE FOR CARRYING OUT THE INVENTION

Hereinafter, the present invention is described in more detail with reference to examples of the fat emulsion of the invention and the pain-relieving fat emulsion of the invention.

### Examples 1 to 10

### Preparation of the fat emulsion of the invention

The fat emulsions of the invention comprising the components shown in Tables 2 and 3 were prepared. The values in each Table represent the proportion of each component in terms of a percentage of weight to volume (w/v%) based on the total volume of the fat emulsion obtained. That is, the proportion of each component is represented by "w/v%" based on the total amount of the fat emulsion before the addition of the local anaesthetic (2% lidocaine hydrochloride) according to the processes of Examples 11 to 20 described later.

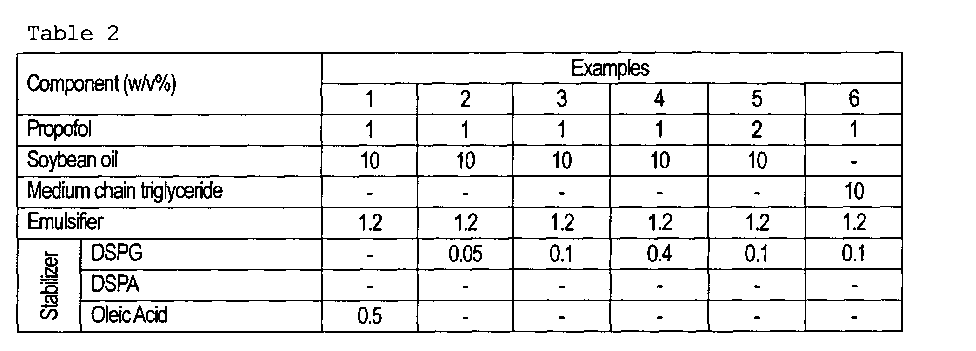

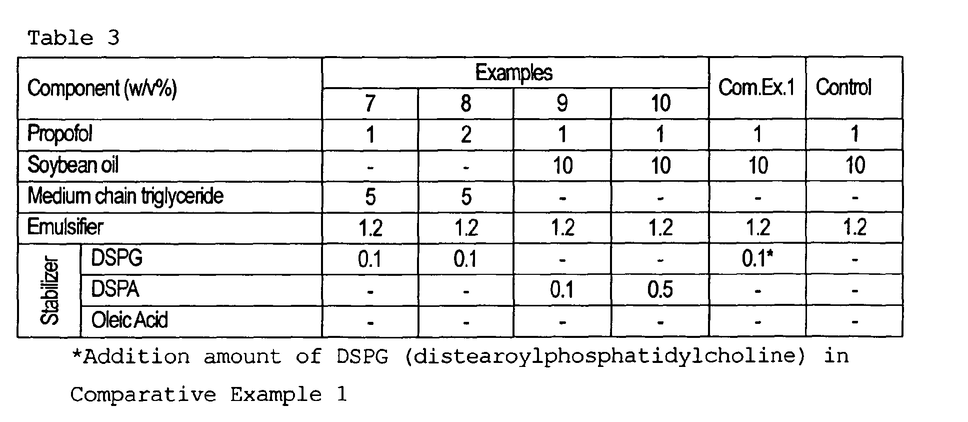

### Components in Tables 2 and 3 are as follows:

Soybean oil: purified soybean oil (manufactured by Nisshin Oil Mills, Ltd.);
Medium chain triglyceride: "Panasate 810" (registered trademark, manufactured by NOF Corporation);
Emulsifier: lecithin (purified egg yolk lecithin, manufactured by Kewpie)
Stabilizer: DSPG: distearoylphosphatidylglycerol
   DSPA: distearoylphosphatidic acid

Among the components shown in Tables 2 and 3, propofol and soybean oil or medium chain triglyceride were mixed. To this mixture were added an emulsifier and a stabilizer, and to this was further added a solution in which glycerol was dissolved in water for injection in such a manner as to yield the final concentration of 2.21%, and the result was heated and roughly emulsified in a nitrogen atmosphere at a rotation rate of 25000 revolutions/minute for 10 minutes using a Polytron™ homogenizer (manufactured by KINEMATICA).

Subsequently, the pH of the obtained roughly emulsified liquid was adjusted to 8.2 to 8.3, and then was subjected to a high-pressure homogeni zer (made byAPV) until the mean particle diameter thereof was 0.3 µm or less at an emulsification temperature of 40°C to 80°C in a nitrogen atmosphere and under an emulsification pressure of 550 kg/cm², yielding a refined emulsion.

10 mL of the obtained emulsion was placed in a 10 mL-glass vial, and it was subj ected to high-pressure steam sterilization (121°C, 12 minutes) with an autoclave after being sealed, yielding a sample of apropofol-containing fat emulsion (the fat emulsion of the invention) comprising a stabilizer.

### Comparative Example 1

### Preparation of a comparative fat emulsion

The procedure of Example 9 was repeated to prepare a sample of a comparative fat emulsion except that the same amount of DSPC (distearoylphosphatidylcholine) was used instead of DSPA (distearoylphosphatidic acid) as a stabilizer.

### Examples 11 to 20

### Preparation of the pain-relieving fat emulsion of the invention

Each of the fat emulsion samples of the invention obtained in Examples 1 to 10 was mixed with 2% lidocaine hydrochloride (trade name: "2% Xylocaine injection", manufactured by AstraZeneca) at a ratio of 9:1 (volume ratio), to prepare samples of the pain-relieving fat emulsion of the invention (Examples 11 to 20). The fat emulsion samples of the invention used in Examples 11 to 20 are the same as those obtained in Examples 1 to 10.

### Comparative Example 2

### Preparation of a comparative pain-relieving fat emulsion

To the comparative fat emulsion sample obtained in Comparative Example 1 was added 2% lidocaine hydrochloride at a ratio of 9:1 (volume ratio), to prepare a comparative fat emulsion sample.

### Test Example 1

### Tests for fat emulsion stability

### (1) Test sample

The following tests were conducted using the pain-relieving fat emulsion samples of the invention prepared in Examples 11 to 20, the comparative pain-relieving fat emulsion sample of Comparative Example 2 and a control pain-relieving fat emulsion sample that was prepared as follows.

The control fat emulsion sample was prepared by mixing 2% lidocaine hydrochloride with a commercially available propofol-containing fat emulsion (trade name: 1% "Diprivan™" injection comprising 1 w/v% of propofol, 10 w/v% of soybean oil, and 1.2 w/v% of an emulsifier, manufactured by AstraZeneca) in such a manner that the ratio of fat emulsion : 2% lidocaine hydrochloride was 9:1 (volume ratio) in the same manner as in Examples 11 to 20.

### (2) Test items

The mean particle diameter of emulsion particles for each sample was measured 3 hours, 6 hours, and 24 hours after the preparation thereof. The mean particle diameter was measured according to a dynamic light scattering method.

The pH of each sample was determined by a pH meter (trade name "HM-30G", manufactured by TOA DKK), which is referred to as ph "after mixing".

The appearance of each sample was visually observed 24 hours after the preparation thereof, and evaluated as follows:
A: No separation of the emulsion into two phases was observed.
B: Separation of the emulsion into two phases was observed.

### (3) Results

The obtained results are shown in Tables 4 and 5.

The term "previous value" in the mean particle diameter item in Tables 4 and 5 represents the mean particle diameter of the emulsion particles of each fat emulsion before the addition of lidocaine hydrochloride. For the measurement, the same method was employed as for the fat emulsion samples in Examples 1 to 10 and Comparative Example 1, and a commercially available propofol-containing fat emulsion. The term "before mixing" in the pH value item represents the pH of each fat emulsion before the mixing of lidocaine hydrochloride.

**Table 4**

| | | Examples | | | | | |
|---|---|---|---|---|---|---|---|
| | | 11 | 12 | 13 | 14 | 15 | 16 |
| Mean particle diameter (µm) | Previous value | 0.21 | 0.21 | 0.22 | 0.25 | 0.20 | 0.20 |
| | 3 hours later | 0.21 | 0.23 | 0.23 | 0.24 | 0.20 | 0.20 |
| | 6 hours later | 0.21 | 0.24 | 0.23 | 0.23 | 0.21 | 0.20 |
| | 24 hours later | 0.21 | 0.24 | 0.22 | 0.24 | 0.21 | 0.20 |
| PH | Before mixing | 8.16 | 7.28 | 7.30 | 7.39 | 7.04 | 6.90 |
| | After mixing | 6.50 | 5.78 | 5.78 | 5.93 | 5.55 | 5.65 |
| Appearance (24 hours later) | | A | A | A | A | A | A |

**Table 5**

| | | Examples | | | | Comp. Ex. | Control |
|---|---|---|---|---|---|---|---|
| | | 17 | 18 | 19 | 20 | 2 | |
| Mean particle diameter (µm) | Previous value | 0.21 | 0.21 | 0.22 | 0.20 | 0.21 | 0.21 |
| | 3 hours later | 0.21 | 0.21 | 0.23 | 0.20 | 0.22 | 0.21 |
| | 6 hours later | 0.21 | 0.22 | 0.24 | 0.21 | 0.22 | 0.22 |
| | 24 hours later | 0.21 | 0.22 | 0.22 | 0.19 | 0.23 | 0.23 |
| PH | Before mixing | 6.80 | 7.15 | 7.48 | 7.60 | 6.92 | 7.85 |
| | After mixing | 5.73 | 5.49 | 5.95 | 6.39 | 5.75 | 6.05 |
| Appearance (24 hours later) | | A | A | A | A | B | B |

### (4) Consideration of the results

As can be seen from the results in Tables 4 and 5, the fat emulsion samples of the invention (Examples 11 to 20) did not separate into two phases 24 hours after the mixing of lidocaine hydrochloride even though the pH value changed almost in the same manner as in the comparative sample of Comparative Example 2. Therefore, it is clear that the fat emulsion samples of the invention have excellent emulsion stability.

The fat emulsion samples of the invention of Examples 1 to 10 were examined under the condition that no lidocaine hydrochloride was added in terms of stability after 6 months' storage at 40°C, which showed that the appearance thereof did not change, and the storage stability was excellent. It was confirmed that this storage stability is noticeable particularly in the fat emulsion samples of the invention of Examples 3 and 7.

The pain-relieving fat emulsion samples of the invention (Examples 11 to 20) tested above were all able to prevent or alleviate pain (vascular pain) arising as side effects during the intravenous administration thereof.

### Examples 21 to 24

### Preparation of a sample of the pain-relieving fat emulsion of the invention

The pain-relieving fat emulsions of the invention comprising the components shown in Table 6 were prepared as follows.

Among the components shown in Table 6, propofol, lidocaine hydrochloride and soybean oil or a medium chain triglyceride were mixed. To this mixture were added an emulsifier and a stabilizer, and to this was further added a solution in which glycerol was dissolved in water for injection in such a manner as to yield the final concentration of 2.21%, and the result was heated and roughly emulsified in a nitrogen atmosphere at a rotation rate of 25000 revolutions/minute for 10 minutes using a Polytron™ homogenizer (manufactured by KINEMATICA).

Subsequently, the pH of the obtained roughly emulsified liquid was adjusted to 8.2 to 8.3, and then was subjected to a high-pressure homogenizer (manufactured by APV) under the conditions of a nitrogen atmosphere, an emulsification temperature of 40°C to 80°C, and an emulsification pressure of 550 kg/cm² until the mean particle diameter thereof was 0.3 µm or less, yielding a refined emulsion.

10 mL of the obtained emulsion was placed in a 10-mL glass vial, and it was subjected to high-pressure steam sterilization (121°C, 12 minutes) with an autoclave after being sealed, yielding a sample of the pain-relieving fat emulsion of the invention.

### Comparative example 3

### Preparation of a comparative pain-relieving fat emulsion

The procedure of Example 21 was repeated, except that DSPG (distearoylphosphatidylglycerol) as a stabilizer was not used, to prepare a sample of a comparative fat emulsion comprising the components of Table 6.

**Table 6**

| Components (w/v%) | Examples | | | | Comp. Ex. |
|---|---|---|---|---|---|
| | 21 | 22 | 23 | 24 | 3 |
| Propofol | 1 | 1 | 1 | 1 | 1 |
| Lidocaine hydrochloride | 0.2 | 0.1 | 0.2 | 0.2 | 0.2 |
| Soybean oil | 10 | 10 | - | - | 10 |
| Medium chain triglyceride | - | - | 10 | 5 | - |
| Emulsifier | 1.2 | 1.2 | 1.2 | 1.2 | 1.2 |
| Stabilizer (DSPG) | 0.1 | 0.1 | 0.1 | 0.1 | - |

The components in Table 6 are the same as those shown in Tables 2 and 3.

### Test Example 2

### Stability test for emulsion before and after sterilization

With regard to the pain-relieving fat emulsion samples of the invention of Examples 21 to 24 and the comparative pain-relieving fat emulsion sample of Comparative Example 3, the mean particle diameter of the emulsion particles of each sample was measured before and after high-pressure steam sterilization, which seriously affects the emulsion stability of each sample, in the same manner as in Test Example 1, and the appearance thereof 24 hours after the preparation of each sample was visually observed in terms of separation of the emulsion into two phases.

The obtained results are shown in Table 7 in the same manner as in Tables 4 and 5.

**Table 7**

| Mean particle diameter (µm) | Examples | | | | Comp. Ex. |
|---|---|---|---|---|---|
| | 21 | 22 | 23 | 24 | 3 |
| Before sterilization | 0.22 | 0.21 | 0.21 | 0.19 | 0.21 |
| After sterilization | 0.21 | 0.21 | 0.22 | 0.21 | 2.29 |
| Appearance (24 hours later) | A | A | A | A | B |

The following can be seen from the results shown in Table 7 with regard to the pain-relieving fat emulsion samples of the invention: the mean particle diameter of the emulsion particles did not substantially change between the states before and after sterilization; the emulsion particles were uniform and fine; and the fat emulsion samples did not separate into two phases. Therefore, it is clear that they have excellent emulsion stability. In contrast, as shown in the Comparative Example 3, in a propofol-containing fat emulsion to which only lidocaine hydrochloride for relieving pain was added, i.e., no stabilizer being added, the emulsion particles thereof agglomerate after sterilization and the emulsion stability sharply reduces, thereby separating the emulsion into two phases.

The appearance of the pain-relieving fat emulsion samples of the invention of Examples 21 to 24 was observed after 6 months' storage at 40°C, which showed that the appearance of the sample did not change. Thus, it was confirmed that the storage stability thereof is maintained over an extended period of time.

The pain-relieving fat emulsion samples of the invention tested above were all able to prevent or alleviate pain (vascular pain) arising as side effects during the intravenous administration thereof.

### Examples 25 to 60

### Preparation of a sample of the pain-relieving fat emulsion of the invention (kit preparation)

Samples of the pain-relieving fat emulsion of the invention comprising the components shown in Tables 8 to 12 were prepared according to the processes of Examples 1 to 10 and Examples 11 to 20.

Among the components shown in each Table, propofol and soybean oil or a medium chain triglyceride were mixed. To this mixture were added an emulsifier and a stabilizer, and to this was further added a solution in which glycerol was dissolved in water for injection in such a manner as to yield the final concentration of 2.21%. The result was heated and roughly emulsified in a nitrogen atmosphere at a rotation rate of 25000 revolutions/minute for 10 minutes using a Polytron™ homogenizer (manufactured by KINEMATICA).

Subsequently, the pH of the obtained roughly emulsified liquid was adjusted to 8.2 to 8.3, and the resulting liquid was then subjected to a high-pressure homogenizer (manufactured by APV) under the conditions of a nitrogen atmosphere, an emulsification temperature of 40°C to 80°C, and an emulsification pressure of 550 kg/cm² until the mean particle diameter thereof was 0. 3 µm or less, yielding a refined emulsion.

10 mL of the obtained emulsion was placed in a 10-mL glass vial, and it was subjected to high-pressure steam sterilization (121°C, 12 minutes) with an autoclave after being sealed, yielding a sample of the fat emulsion of the invention.

Each of the fat emulsion samples of the invention obtained above (Examples 25 to 52) was mixed with 2% lidocaine hydrochloride (trade name: "2% Xylocaine injection", manufactured by AstraZeneca, which is listed as "lidocaine" in the Tables) at a ratio of 9:1 (volume ratio), thereby preparing a sample of the pain-relieving fat emulsion of the invention which contains 0.2% of lidocaine.

To each of the fat emulsion samples of the invention obtained above (Examples 53 and 54) was added lidocaine hydrochloride while varying the addition amount, to prepare two types of the pain-relieving fat emulsion of the invention, one of which contains 0.01% of lidocaine hydrochloride (mixed volume ratio = 199:1) and another of which contains 0.3% of lidocaine hydrochloride (mixed volume ratio = 17:3).

Each of the fat emulsion samples of the invention obtained above (Examples 55 to 60) was mixed with 0.2% of mepivacaine, 0.05% of bupivacaine, 0.02% of ropivacaine, 0.03% of dibucaine, 0.2% of procaine, and 0.05% of tetracaine, yielding 6 types of the pain-relieving fat emulsion of the invention.

Each sample obtained was measured in terms of the mean particle diameter of the emulsion particles, the pH, and the visually-observed appearance 24 hours after the samples were prepared, in the same manner as in Test Example 1, and the evaluation results are shown in Tables 8 to 12 in the same manner as in Tables 4 and 5.

In Tables 8 to 12, the amount of each component excluding a local anaesthetic is shown as w/v% in terms of the composition before the mixing of the local anaesthetic. The amount of the local anaesthetic is shown as w/v% based on the total amount of the composition after the mixing of the same. In Tables 8 to 12, the components are the same as those shown in Tables 2 and 3, or as follows:
Stabilizers:
   - DSPS: :distearoylphosphatidylserine
   - DDPG: :didecanoylphosphatidylglycerol
   - DMPG: :dimyristoylphosphatidylglycerol
   - DOPG: :dioleoylphosphatidylglycerol
   - DSPE-PEG: :distearoylphosphatidylethanolamine polyethylene glycol 2000
   (trade name, "SUNBRIGHT DSPE-020CN", manufactured by NOF CORPORATION)

DSPE-PEG is a compound according to General Formula (I), wherein R₁ and R₂ each represent a stearoyl group, R₃ represents a methyl group, R₄ represents a hydrogen atom, and X represents a -CO- group, and the average molecular weight is 2000.

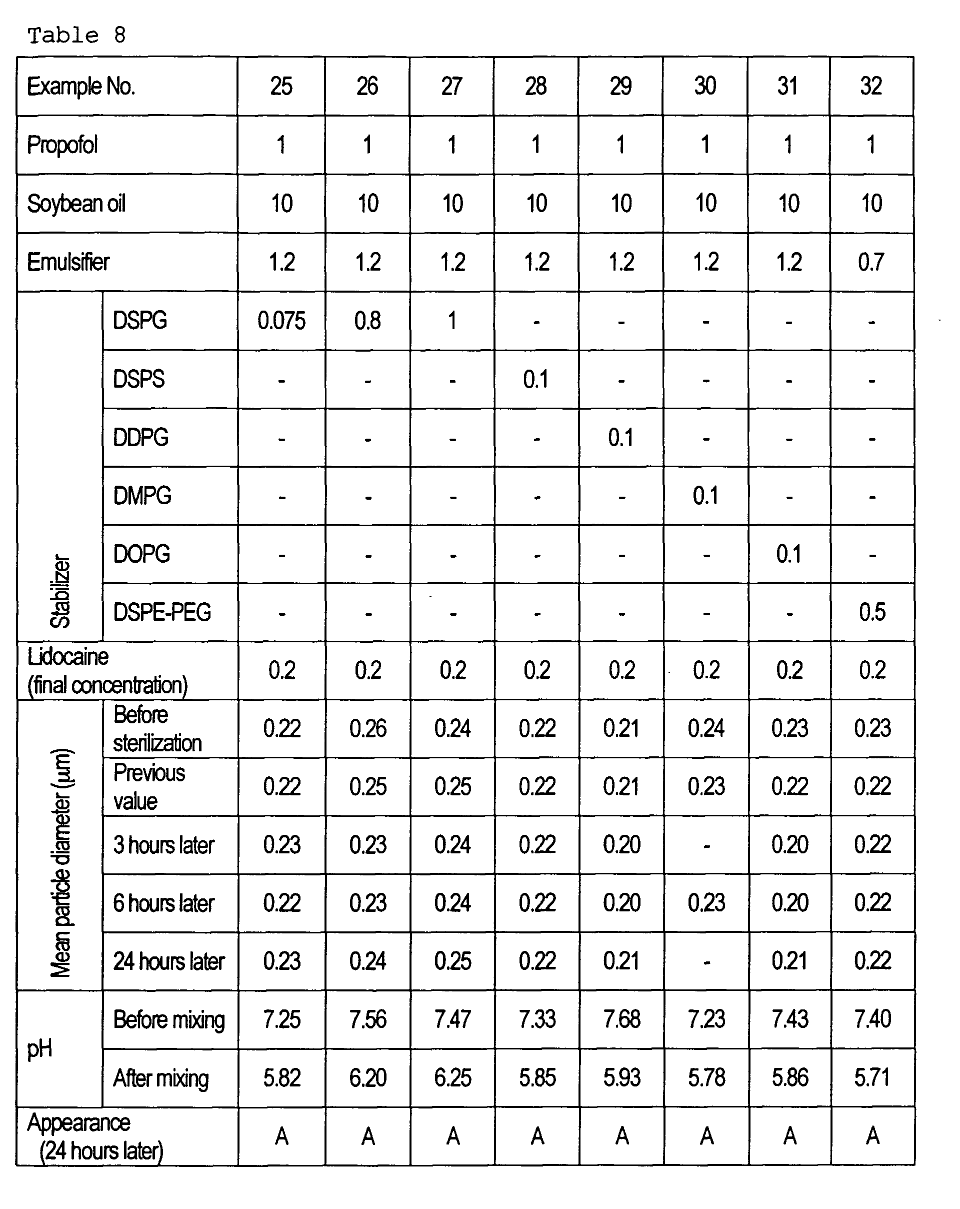

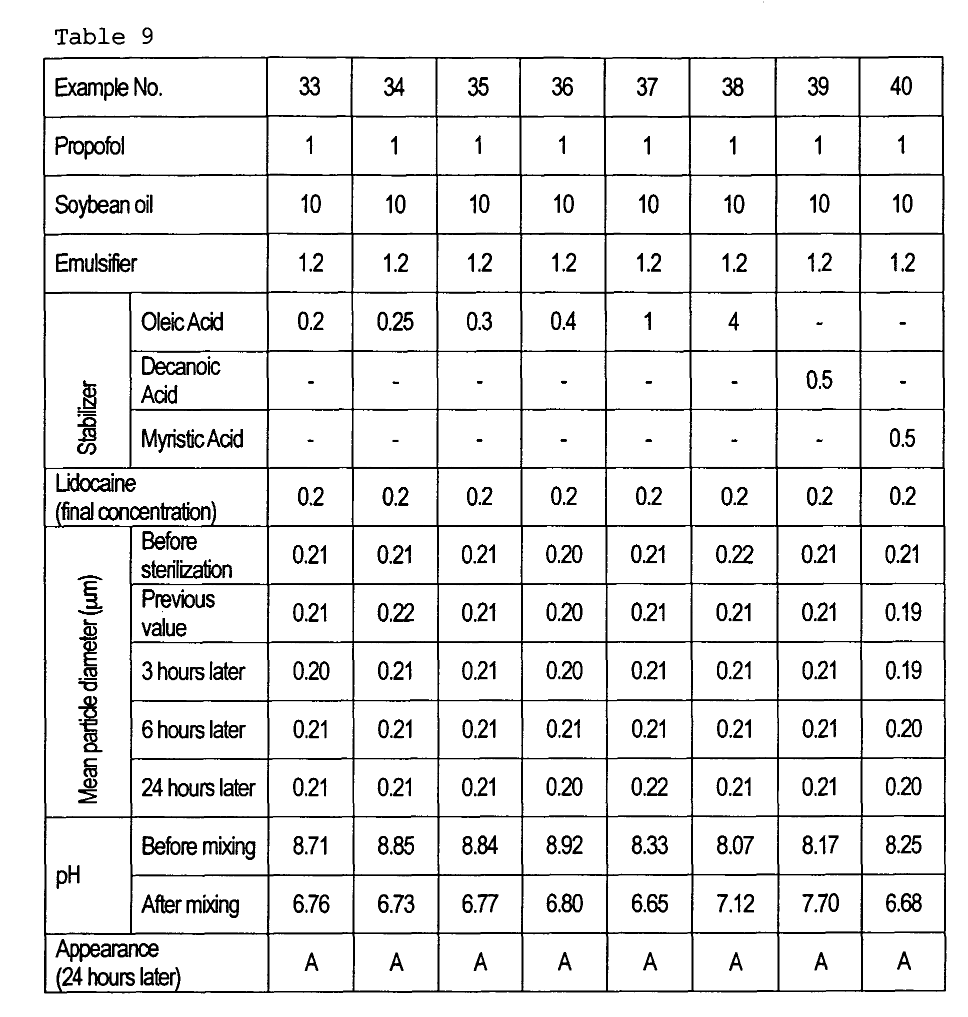

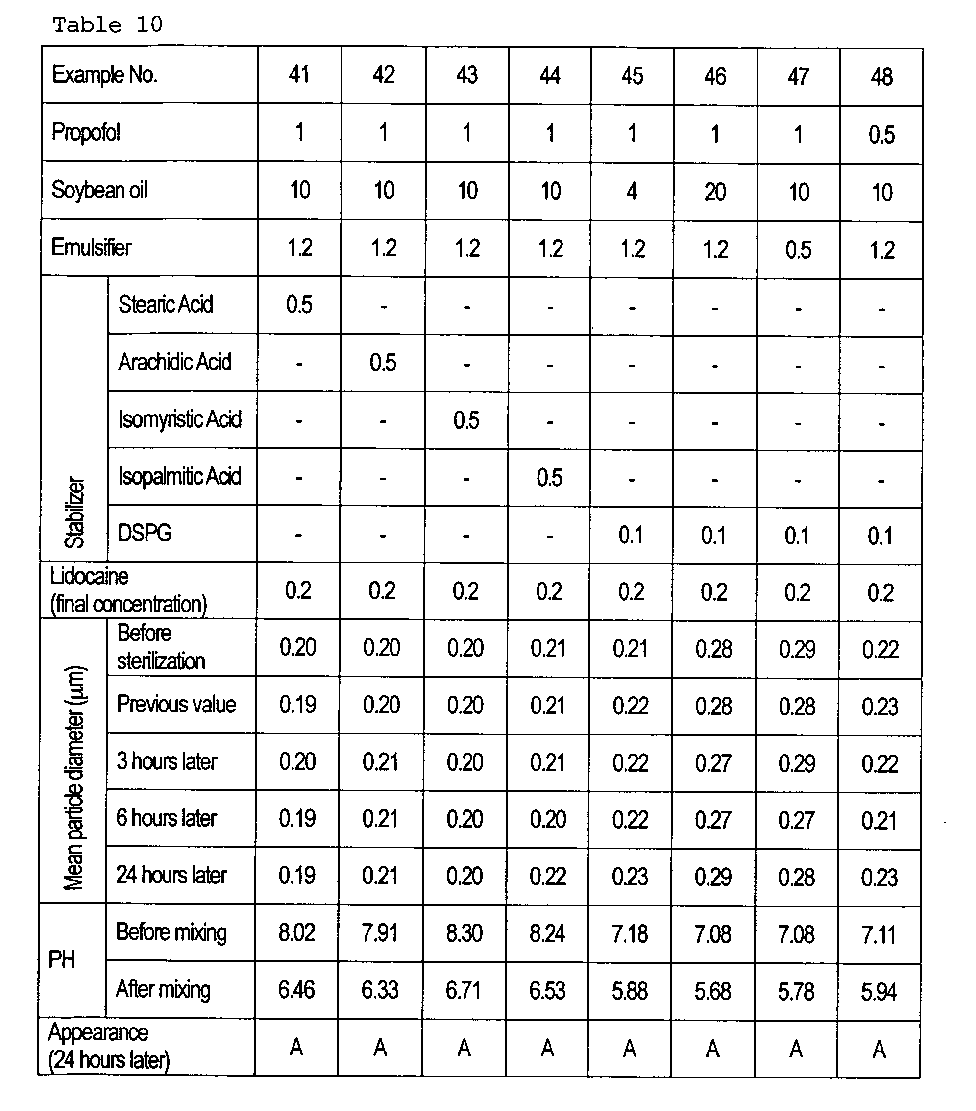

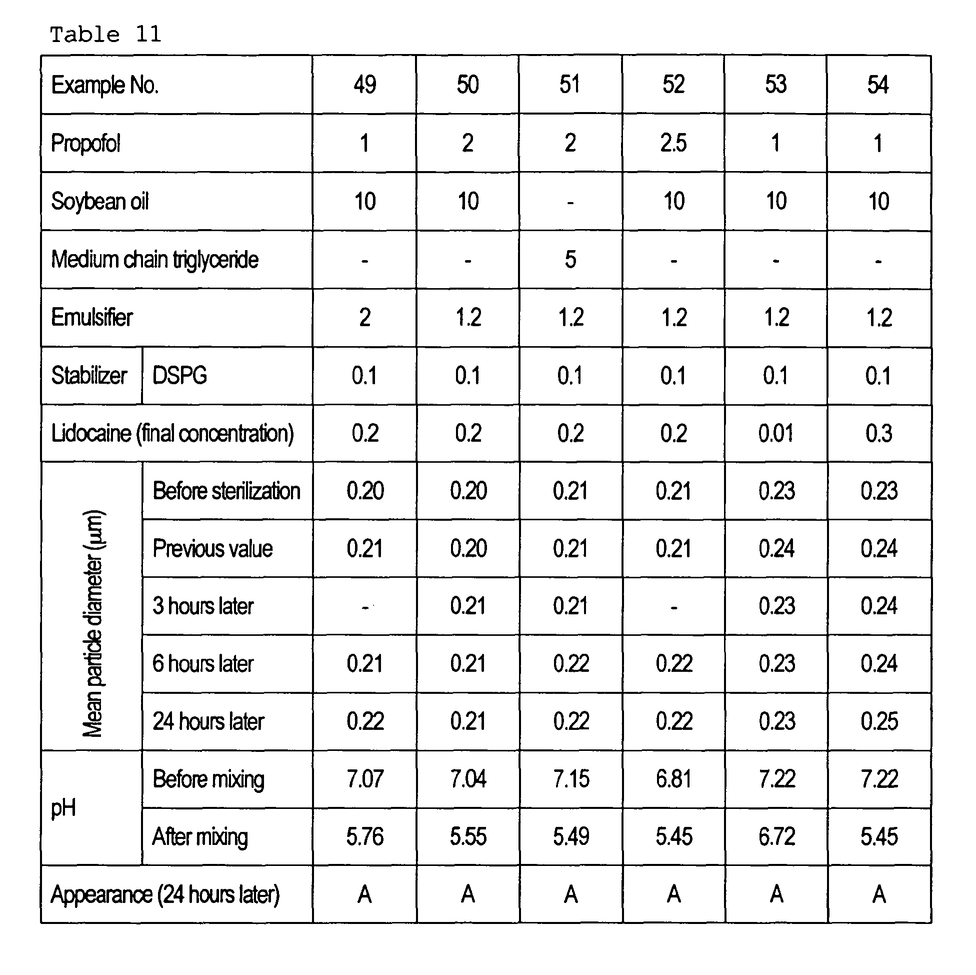

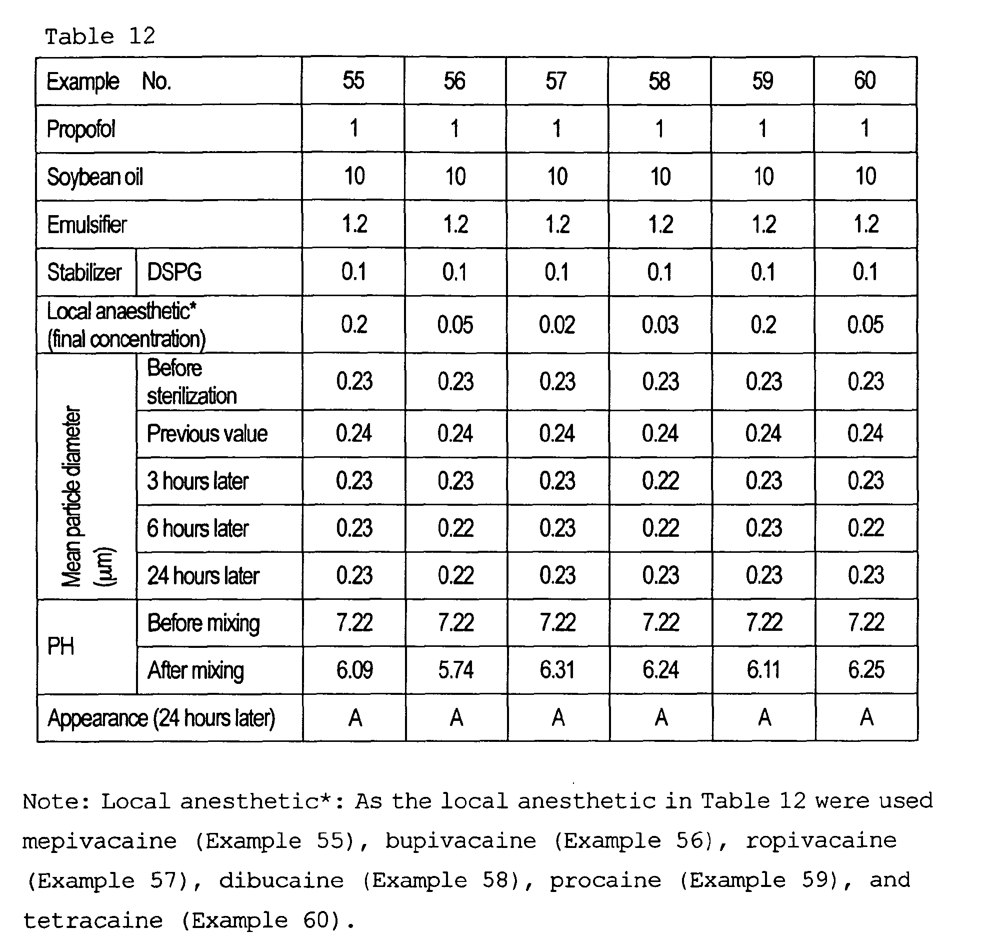

As can be seen from the results shown in Tables 8 to 12, every pain-relieving fat emulsion of the invention has excellent emulsion stability because the mean particle diameter of the emulsion particles did not increase and the emulsion did not separate into two phases.

### Examples 61 to 89

### Preparation of a sample of the pain-relieving fat emulsion of the invention (premix formulation)

Pain-relieving fat emulsions of the invention comprising the components shown in Tables 13 to 16 were prepared in the same manner as in Examples 21 to 24.

In Examples 85 to 89, the following local anaesthetics were used in place of lidocaine hydrochloride: mepivacaine (Example 85), bupivacaine (Example 86), dibucaine (Example 87), procaine (Example 88), and tetracaine (Example 89) (referred to as "local anaesthetic*" in the tables).

The pain-relieving fat emulsion samples of the invention obtained above were tested in terms of maintained emulsion stability before and after sterilization in the same manner as in Test Example 2.

The obtained results (the mean particle diameter of emulsion particles before and after sterilization) are shown in Tables 13 to 16 in the same manner as in Tables 4 and 5.

In each table, the amount of each of the components is an amount based on the total amount of the composition (w/v%), and is shown as w/v%. The components of each table are the same as those shown in Tables 2, 3, and 8.

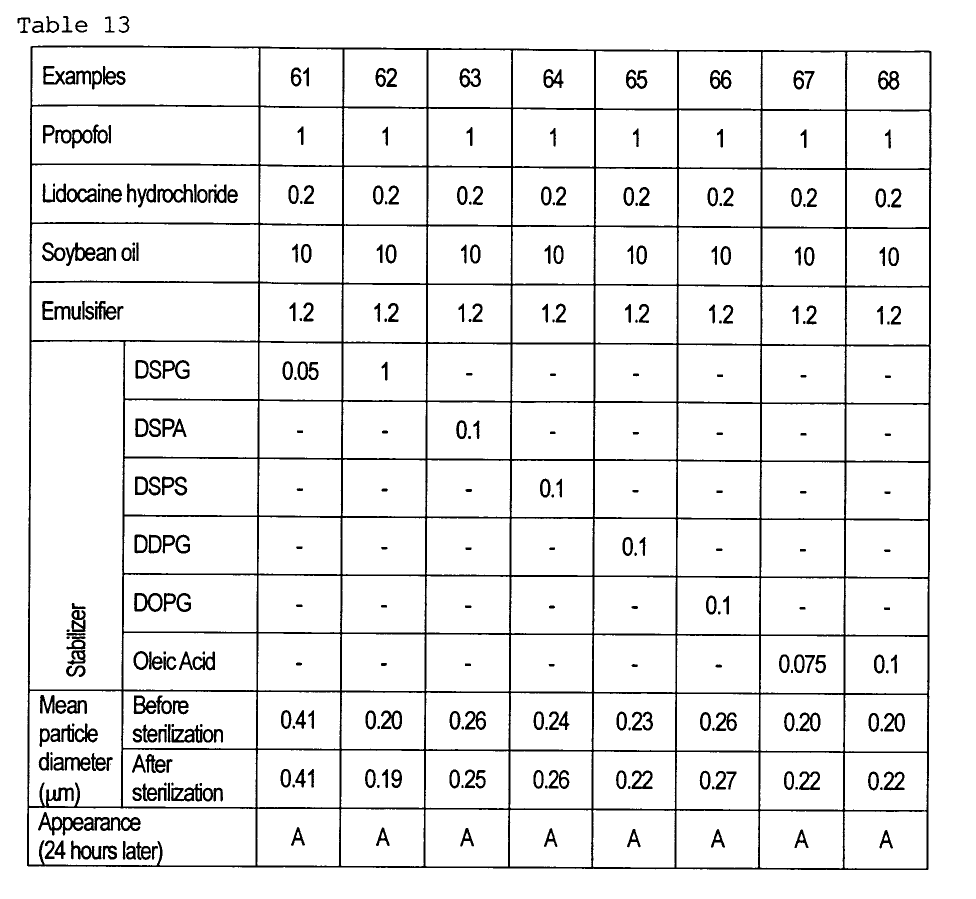

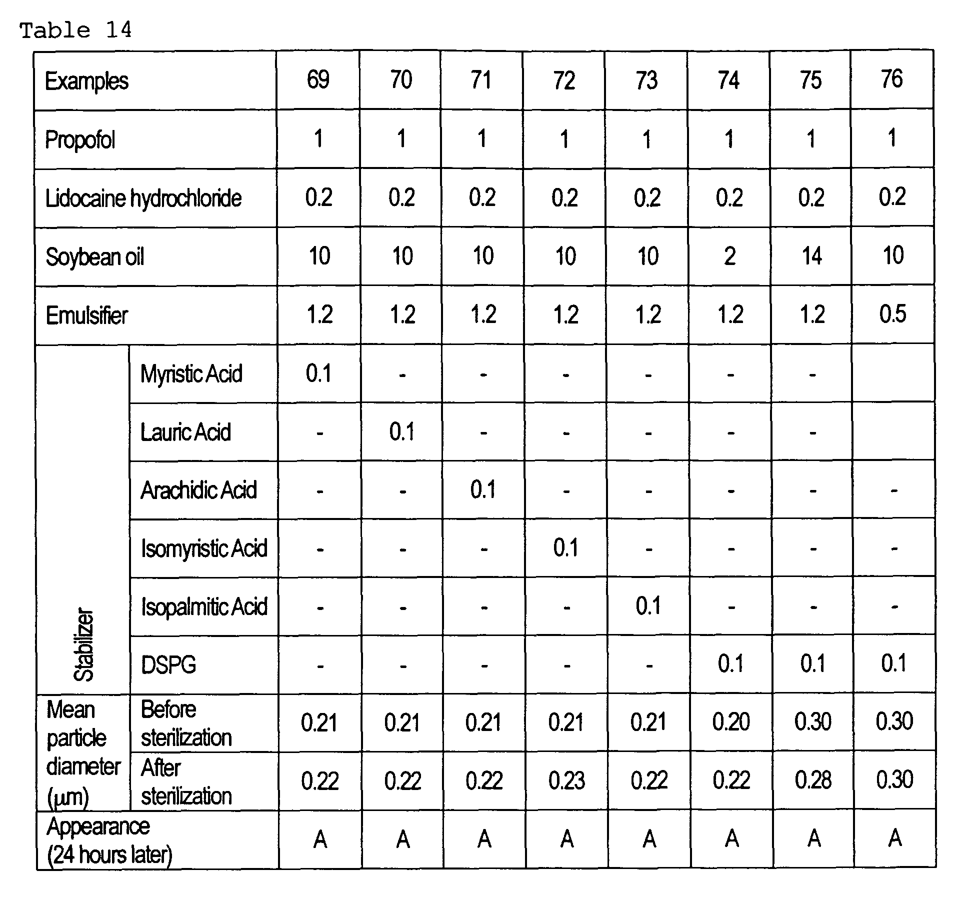

**Table 15**

| | | | | | | | | | |
|---|---|---|---|---|---|---|---|---|---|
| Examples | | 77 | 78 | 79 | 80 | 81 | 82 | 83 | 84 |
| Propofol | | 1 | 1 | 1 | 0.5 | 1.5 | 1 | 1 | 1 |
| Soybean oil | | - | - | 10 | 10 | 10 | 10 | 10 | 10 |
| Medium chain triglyceride | | 10 | 5 | - | - | - | - | - | - |
| Emulsifier | | 1.2 | 1.2 | 2 | 1.2 | 1.2 | 1.2 | 1.2 | 1.2 |
| Stabilizer | DSPG | 0.1 | 0.1 | 0.1 | 0.1 | 0.1 | 0.1 | 0.1 | 0.1 |
| Local anaesthetic | Lidocaine hydrochloride | 0.2 | 0.2 | 0.2 | 0.2 | 0.2 | 0.01 | 0.1 | 0.3 |
| Mean particle diameter (µm) | Before sterilization | 0.21 | 019 | 0.26 | 0.24 | 0.29 | 0.24 | 0.21 | 0.35 |
| | After sterilization | 0.22 | 0.21 | 0.28 | 0.23 | 0.29 | 0.23 | 0.21 | 0.37 |
| Appearance (24 hours later) | | A | A | A | A | A | A | A | A |

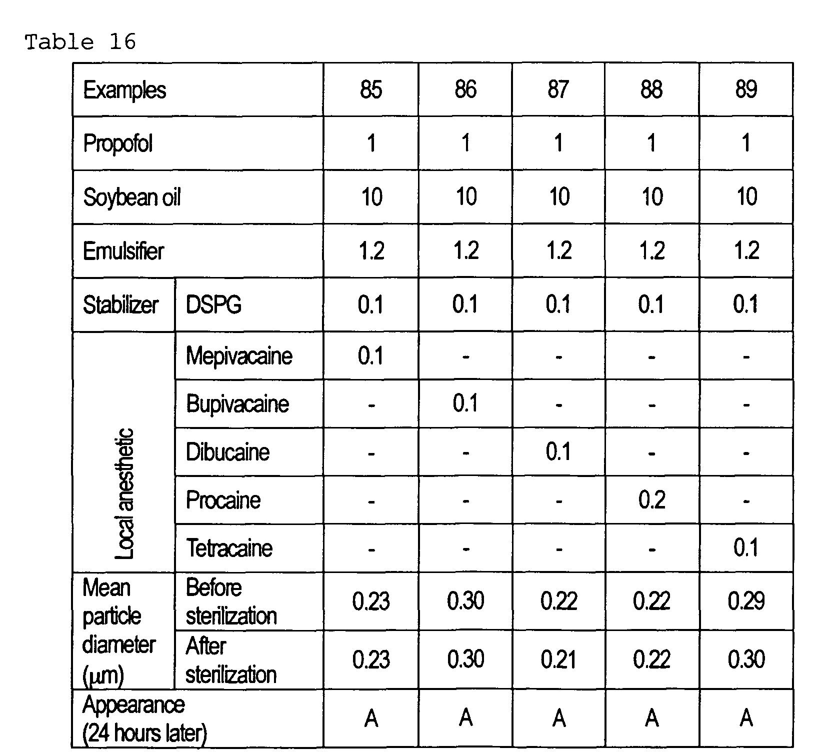

The following can be seen from the results shown in Tables 13 to 16 with regard to the pain-relieving fat emulsion samples of the invention: the mean particle diameter of the emulsion particles did not substantially change between the states before and after sterilization; the emulsion particles were uniform and fine; and the fat emulsion samples did not separate into two phases. Therefore, it is clear that they have excellent emulsion stability.

The appearance of the pain-relieving fat emulsion samples of the invention was observed after 6 months' storage at 40°C, which showed that the appearance thereof did not change. It was thus confirmed that the samples exhibit extended storage stability. The pain-relieving fat emulsion samples of the invention tested above were able to prevent or alleviate pain arising as side effects of the intravenous administration thereof.

As discussed above in detail, the present invention provides a propofol-containing fat emulsion with improved emulsion stability, to which a local anaesthetic, such as lidocaine, etc., can be added for preventing or mediating side effects, i.e., pain (vascular pain) arising during the (intravenous) administration of the emulsion and which comprises a specific stabilizer for avoiding reduced emulsion stability caused by the addition of the local anaesthetic.

The invention also provides a stable propofol-containing fat emulsion which produces no pain when administered. The fat emulsion further comprises the local anaesthetic, such as lidocaine as a soothing agent.

The fat emulsion of the invention has excellent emulsion stability and high safety. The excellent emulsion stability of the fat emulsion can be obtained without adjusting the pH thereof, is not impaired even when the fat emulsion is subjected to heat sterilization, and can be maintained for an extended period of time.

## Claims

1. A fat emulsion with which a local anaesthetic is mixed before use, and which comprises propofol, an oily component, and an emulsifier, the fat emulsion further comprising a stabilizer selected from the following (a), (b), (c), or (d):
(a) at least one phospholipid selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid;
(b) at least one phospholipid derivative selected from phosphatidylethanolamines modified with polyalkyleneglycol, wherein a fatty acid esterified to a glycerol moiety is a C₁₀₋₂₂ linear or branched, saturated or unsaturated fatty acid;
(c) at least one fatty acid selected from the group consisting of C₁₀₋₂₂ linear or branched, saturated or unsaturated fatty acids; or
(d) a mixture of at least two members selected from the above groups (a), (b), and (C), wherein
the stabilizer (a), (b), or (c) is present at a concentration of 0.01 to 1 w/v%, 0.01 to 1 w/v%, 0.05 to 5 w/v%, respectively, per the total amount of fat emulsion and local anaesthetic to be mixed therewith before use.

2. The fat emulsion according to Claim 1, wherein
(1) propofol is present at a concentration of 0.4 to 5 w/v%,
(2) an oily component is present at a concentration of 2 to 20 w/v%, and
(3) an emulsifier is present at a concentration of 0.4 to 5 w/v%, per the total amount of fat emulsion and local anaesthetic to be mixed therewith before use.

3. The fat emulsion according to Claim 1, wherein a local anaesthetic is at least one member selected from the group consisting of lidocaine, mepivacaine, bupivacaine, ropivacaine, dibucaine, procaine, procaine chloride, tetracaine and pharmacologically acceptable acid addition salts thereof.

4. The fat emulsion according to Claim 1, wherein the local anaesthetic is present at a concentration of 0.01 to 1 w/v%, per the total amount of fat emulsion and local anaesthetic to be mixed therewith before use.

5. The fat emulsion according to Claim 1, wherein the stabilizer is at least one phospholipid (a) selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid.

6. The fat emulsion according to Claim 1, wherein the stabilizer is at least one phospholipid (a) selected from the group consisting of phosphatidylglycerol, phosphatidic acid, phosphatidylinositol, and phosphatidylserine wherein a fatty acid esterified to a glycerol moiety is a C₁₂₋₁₈ linear or branched, saturated or unsaturated fatty acid.

7. The fat emulsion according to Claim 1, wherein the stabilizer is at least one phospholipid (a) selected from the group consisting of distearoylphosphatidylglycerol, dipalmitoylphosphatidylglycerol, dioleoylphosphatidylglycerol, distearoylphosphatidic acid, dipalmitoylphosphatidic acid, dioleoylphosphatidic acid, distearoylphosphatidylinositol, dipalmitoylphosphatidylinositol, dioleoylphosphatidylinositol, distearoyphosphatidylserine, dipalmitoylphosphatidylserine, and dioleoylphosphatidylserine.

8. The fat emulsion according to Claim 5, wherein the stabilizer is present at a concentration of 0.03 to 1 w/v%, per the total amount of fat emulsion and local anaesthetic to be mixed therewith before use.

9. The fat emulsion according to Claim 1, wherein the stabilizer is at least one phospholipid derivative (b) selected from phosphatidylethanolamines modified with polyalkyleneglycol, wherein a fatty acid esterified to a glycerol moiety is a C₁₀₋₂₂ linear or branched, saturated or unsaturated fatty acid.

10. The fat emulsion according to Claim 1, wherein the stabilizer is at least one phospholipid derivative (b) selected from phosphatidylethanolamines modified with polyalkyleneglycol having an average molecular weight of 1000 to 5000, wherein a fatty acid esterified to a glycerol moiety is a C₁₄₋₁₈ linear or branched, saturated or unsaturated fatty acid.

11. The fat emulsion according to Claim 1, wherein the stabilizer is at least one phospholipid derivative (b) selected from the group consisting of distearoylphosphatidylethanolamine-polyethylene glycol 5000, distearoylphosphatidylethanolamine-polyethylene glycol 3000, and distearoylphosphatidylethanolamine-polyethylene glycol 2000.

12. The fat emulsion according to Claim 9, wherein the stabilizer is present at a concentration of 0.1 to 1 w/v%, per the total amount of fat emulsion and local anaesthetic to be mixed therewith before use.

13. The fat emulsion according to Claim 1, wherein the stabilizer is at least one fatty acid (c) selected from the group consisting of C₁₀₋₂₂ linear or branched, saturated or unsaturated fatty acids.

14. The fat emulsion according to Claim 1, wherein the stabilizer is at least one fatty acid (c) selected from the group consisting of C₁₀₋₂₀ linear or branched, saturated or unsaturated acids.

15. The fat emulsion according to Claim 1, wherein the stabilizer is at least one fatty acid (c) selected from the group consisting of decanoic acid, lauric acid, myristic acid, palmitic acid, stearic acid, arachidic acid, isomyristic acid, isopalmitic acid, and oleic acid.

16. The fat emulsion according to Claim 13, wherein the stabilizer is present at a concentration of 0.1 to 5 w/v%, per the total amount of fat emulsion and local anaesthetic to be mixed therewith before use.

17. A fat emulsion containing container having a multi-compartment that is divided with a partition in such a manner as to allow the compartments to communicate with one another, which container comprises one compartment containing the fat emulsion according to Claim 1 and another compartment containing a local anaesthetic.

18. A pain-relieving fat emulsion comprising propofol, an oily component, an emulsifier, a stabilizer, and a local anaesthetic, wherein the stabilizer is selected from the following (a), (b), (c), or (d):
(a) at least one phospholipid selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid;
(b) at least one phospholipid derivative selected from phosphatidylethanolamines modified with polyalkyleneglycol, wherein a fatty acid esterified to a glycerol moiety is a C₁₀₋₂₂ linear or branched, saturated or unsaturated fatty acid;
(c) at least one fatty acid selected from the group consisting of C₁₀₋₂₂ linear or branched, saturated or unsaturated fatty acids; or
(d) a mixture of at least two members selected from the above groups (a), (b), and (C), wherein
the stabilizer (a), (b), or (c) is present at a concentration of 0.01 to 1 w/v%, 0.01 to 1 w/v%, and 0.05 to 5 w/v%, respectively, in the fat emulsion.

19. The pain-relieving fat emulsion according to Claim 18, wherein
(1) propofol is present at a concentration of 0.4 to 5 w/v%,
(2) an oily component is present at a concentration of 2 to 20 w/v%,
(3) an emulsifier is present at a concentration of 0.4 to 5 w/v%, and
(4) a local anaesthetic is present at a concentration of 0.01 to 1 w/v%, in the fat emulsion.

20. The pain-relieving fat emulsion according to Claim 18, wherein a local anaesthetic is at least one member selected from the group consisting of lidocaine, mepivacaine, bupivacaine, ropivacaine, dibucaine, procaine, procaine chloride, tetracaine and pharmacologically acceptable acid addition salts thereof.

21. The pain-relieving fat emulsion according to Claim 18, wherein the stabilizer is at least one phospholipid (a) selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid.

22. The pain-relieving fat emulsion according to Claim 18, wherein the stabilizer is at least one phospholipid (a) selected from the group consisting of phosphatidylglycerol, phosphatidic acid, phosphatidylinositol, and phosphatidylserine wherein a fatty acid esterified to a glycerol moiety is a C₁₂₋₁₈ linear or branched, saturated or unsaturated fatty acid.

23. The pain-relieving fat emulsion according to Claim 18, wherein the stabilizer is at least one phospholipid (a) selected from the group consisting of distearoylphosphatidylglycerol, dipalmitoylphosphatidylglycerol, dioleoylphosphatidylglycerol, distearoylphosphatidic acid, dipalmitoylphosphatidic acid, dioleoylphosphatidic acid, distearoylphosphatidylinositol, dipalmitoylphosphatidylinositol, dioleoylphosphatidylinositol, distearoylphosphatidylserine, dipalmitoylphosphatidylserine, and dioleoylphosphatidylserine.

24. The pain-relieving fat emulsion according to Claim 21, wherein the stabilizer is present at a concentration of 0.03 to 1 w/v% in the fat emulsion.

25. The pain-relieving fat emulsion according to Claim 18, wherein the stabilizer is at least one phospholipid derivative (b) selected from phosphatidylethanolamines modified with polyalkyleneglycol, wherein a fatty acid esterified to a glycerol moiety is a C₁₀₋₂₂ linear or branched, saturated or unsaturated fatty acid.

26. The pain-relieving fat emulsion according to Claim 18, wherein the stabilizer is at least one phospholipid derivative (b) selected from phosphatidylethanolamines modified with polyalkyleneglycol having the average molecular weight of 1000 to 5000, wherein a fatty acid esterified to a glycerol moiety is a C₁₄₋₁₈ linear or branched, saturated or unsaturated fatty acid.

27. The pain-relieving fat emulsion according to Claim 18, wherein the stabilizer is at least one phospholipid derivative (b) selected from the group consisting of distearoylphosphatidylethanolamine-polyethylene glycol 5000, distearoylphosphatidylethanolamine-polyethylene glycol 3000, and distearoylphosphatidylethanolamine-polyethylene glycol 2000.

28. The pain-relieving fat emulsion according to Claim 25, wherein the stabilizer is present at a concentration of 0.1 to 1 w/v% in the fat emulsion.

29. The pain-relieving fat emulsion according to Claim 18, wherein the stabilizer is at least one fatty acid (c) selected from the group consisting of C₁₀₋₂₂ linear or branched, saturated or unsaturated fatty acids.

30. The pain-relieving fat emulsion according to Claim 18, wherein the stabilizer is at least one fatty acid (c) selected from the group consisting of C₁₀₋₂₀ linear or branched, saturated or unsaturated fatty acids.

31. The pain-relieving fat emulsion according to Claim 18, wherein the stabilizer is at least one fatty acid (c) selected from the group consisting of oleic acid, isomyristic acid, isopalmitic acid, decanoic acid, lauric acid, myristic acid, palmitic acid, stearic acid, and arachidic acid.

32. The pain-relieving fat emulsion according to Claim 29, wherein the stabilizer is present at a concentration of 0.05 to 0.2 w/v% in the fat emulsion.

33. A method for manufacturing a pain-relieving fat emulsion, the method comprising:
mixing a local anaesthetic with a fat emulsion comprising propofol, an oily component, an emulsifier, and a stabilizer, wherein the stabilizer is selected from the following (a), (b), (c), or (d) :
(a) at least one phospholipid selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid;
(b) at least one phospholipid derivative selected from phosphatidylethanolamines modified with polyalkyleneglycol, wherein a fatty acid esterified to a glycerol moiety is a C₁₀₋₂₂ linear or branched, saturated or unsaturated fatty acid;
(c) at least one fatty acid selected from the group consisting of C₁₀₋₂₂ linear or branched, saturated or unsaturated fatty acids; or
(d) a mixture of at least two members selected from the above groups (a), (b), and (c), to thereby obtain a fat emulsion, wherein
the stabilizer (a), (b), or (c) is present at a concentration of 0.01 to 1 w/v%, 0.01 to 1 w/v%, and 0.05 to 5 w/v%, respectively, in the fat emulsion.

34. Use of the following stabilizers (a) to (d) for stabilizing a fat emulsion with which a local anaesthetic is mixed before use and which comprises propofol, an oily component, and an emulsifier, or a pain-relieving fat emulsion which comprises propofol, an oily component, an emulsifier, and a local anaesthetic:
(a) at least one phospholipid selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid;
(b) at least one phospholipid derivative selected from phosphatidylethanolamines modified with polyalkyleneglycol, wherein a fatty acid esterified to a glycerol moiety is a C₁₀₋₂₂ linear or branched, saturated or unsaturated fatty acid;
(c) at least one fatty acid (c) selected from the group consisting of C₁₀₋₂₂ linear or branched, saturated or unsaturated fatty acids; or
(d) a mixture of at least two members selected from the above groups (a), (b), and (c).
